# EUROPÄISCHE PATENTANMELDUNG

(11) **EP 1 923 382 A1**
(43) Veröffentlichungstag der Anmeldung: **21.05.2008**
(21) Anmeldenummer: 06090211.1
(22) Anmeldetag: 18.11.2006
(51) Int. Cl.: C07C 229/24, C07B 59/00, C07C 251/24, C07C 309/73, A61K 51/04, A61K 31/195

(54) **[F-18] markierte L-Glutaminsäure, [F-18] markiertes Glutamin, ihre Derivate und ihre Verwendung sowie Verfahren zu ihrer Herstellung**

(71) Anmelder: Bayer Schering Pharma Aktiengesellschaft, 13353 Berlin (DE)
(72) Erfinder: Dinkelborg, Ludger, 13465 Berlin (DE); Friebe, Matthias, 13509 Berlin (DE); Krasikowa, Raisa Nikolaevna, 197341 St-Petersburg (RU); Belokon, Yuri, 109240 Moscow (RU); Kuznetsova, Olga Fedorovna, 195427 St-Petersburg (RU); Graham, Keith, 10115 Berlin (DE); Lehmann, Lutz, 13357 Berlin (DE); Berndt, Mathias, 12163 Berlin (DE)

(57) **Zusammenfassung**

Es werden die Verbindungen und die Synthese von [F-18] markierter L-Glutaminsäure, [F-18] markiertem L-Glutamat, ihren Derivaten gemäß Formel (I) und ihre Verwendungen beschrieben.

## Beschreibung

Die Erfindung betrifft die in den Patentansprüchen gekennzeichneten Gegenstände, nämlich [F-18] markierte L-Glutaminsäure und [F-18] markiertes L-Glutamin der allgemeinen Formel I, ihre Derivate, sowie ihre Verwendung und Verfahren zu ihrer Herstellung.

Die frühe Diagnose von malignen Tumorerkrankungen spielt eine sehr wichtige Rolle für die Überlebensprognose eines Tumorpatienten. Bei dieser Diagnose sind nicht-invasive, bildgebende diagnostische Verfahren ein wichtiges Hilfsmittel. In den letzten Jahren hat sich hier vor allem die PET-Technologie (Positron-Emissions-Tomographie) als besonders nützlich erwiesen. Die Sensitivität und Spezifität der PET-Technologie hängt wesentlich von der verwendeten signalgebenden Substanz (Tracer) und ihrer Verteilung im Körper ab. Bei der Suche nach geeigneten Tracern versucht man bestimmte Eigenschaften von Tumoren auszunutzen, die Tumorgewebe von gesundem umliegenden Gewebe unterscheiden. Das bevorzugte kommerziell genutzte Isotop, welches für PET Anwendung findet, ist ¹⁸F. ¹⁸F stellt durch seine kurze Halbwertszeit von unter 2 Stunden besondere Anforderungen an die Herstellung geeigneter Tracer. Aufwändige, lange Synthesewege und Aufreinigungen sind mit diesem Isotop nicht möglich, da sonst ein erheblicher Teil der Radioaktivität des Isotops bereits abgeklungen ist, bevor der Tracer zur Diagnose eingesetzt werden kann. Es ist deshalb häufig nicht möglich etablierte Synthesewege für nichtradioaktive Fluorierungen auf die Synthese von ¹⁸F-Tracern anzuwenden. Des weiteren führt die hohe spezifische Aktivität des ¹⁸F (ca. 80 GBq/nmol) zu sehr niedrigen Substanzmengen an [¹⁸F]Fluorid für die Tracersynthese, was wiederum einen extremen Überschuß an Präkursor bedingt und den Erfolg einer auf nichtradioaktiven Fluorierungsreaktionen basierender Radiosynthese-strategie unvorhersehbar gestaltet.

FDG ([¹⁸F]2-Fluorodesoxyglukose)-PET ist ein weithin akzeptiertes und verbreitetes Hilfsmittel in der Diagnose und weiteren klinischen Verfolgung von Tumorerkrankungen. Maligne Tumore konkurrieren mit dem Wirtsorganismus um Glukoseversorgung zur Nährstoffversorgung (Warburg O. Über den Stoffwechsel der Carcinomzelle. Biochem. Zeitschrift 1924; 152: 309-339; Kellof G. Progress and Promise of FDG-PET Imaging for Cancer Patient Management and Oncologic Drug Development. Clin Cancer Res. 2005; 11 (8): 2785-2807) Dabei haben Tumorzellen im Vergleich zu umliegenden Zellen des Normalgewebes gewöhnlich einen erhöhten Glukosestoffwechsel. Dies wird bei der Verwendung von Fluorodesoxyglukose (FDG), einem Glukosederivat genutzt, welches verstärkt in die Zellen transportiert wird, dort aber nach der Phosphorylierung als FDG-6-phosphat metabolisch eingeschlossen wird ("Warburg-Effekt"). ¹⁸F markiertes FDG ist daher ein wirkungsvoller Tracer zur Detektion von Tumorerkrankungen beim Patienten mittels der PET-Technologie. Auf der Suche nach neuen PET Tracern wurden in jüngster Zeit auch zunehmend Aminosäuren für die ¹⁸F PET Bildgebung eingesetzt (z. B. (review): Eur J Nucl Med Mol Imaging. 2002 May;29(5):681-90). Dabei eignen sich einige der ¹⁸F markierten Aminosäuren für die Messung der Geschwindigkeitsrate der Proteinsynthese, die meisten anderen Derivate aber für die Messung der direkten Zellaufnahme im Tumor. Bekannte ¹⁸F markierte Aminosäuren sind z. B. von Tyrosin-, Phenylalanin-, Prolin-, Asparagin- und unnatürlichen Aminosäuren abgeleitet (z. B. J. Nucl Med 1991; 32:1338-1346, J Nucl Med 1996; 37:320-325, J Nucl Med 2001;42:752-754 bzw. J Nucl Med 1999; 40:331-338.). Glutaminsäure und Glutamin sind als ¹⁸F markierte Derivate nicht bekannt, wohingegen nichtradioaktive fluorierte Glutamin- und Glutaminsäurederivate geläufig sind; so z. Bsp. jene, die an γ-Position (z. Bsp. (review): Amino Acids. (2003) Apr;24(3):245-61) oder an β-Position (z. Bsp. Tetrahedron Lett.; 30; 14; 1989; 1799-1802, J. Org. Chem.; 54; 2; 1989; 498-500, Tetrahedron: Asymmetry; 12; 9; 2001; 1303 - 1312) Fluor aufweisen.

Von Glutaminsäurederivaten, die an den chemischen Funktionalitäten Schutzgruppen und in β oder γ Position eine Abgangsgruppe aufweisen, ist in der Vergangenheit bereits berichtet worden. So wurde über Glutamat als Mesylat bzw. Bromid in γ-Position informiert, deren Säure- und Aminfunktionen mit Ester- bzw. Z-Schutzgruppen versehen waren (J. Chem. Soc. Perkin Trans. 1; 1986; 1323-1328) oder beispielsweise von γ-Chloro-Glutaminsäure ohne Schutzgruppen (Synthesis; (1973); 44-46). Über ähnliche Derivate, bei denen aber die Fluchtgruppe in β-Stellung positioniert ist, wurde ebenfalls verschiedentlich berichtet: z. Bsp. Chem. Pharm. Bull.; 17; 5; (1969); 879-885, J.Gen.Chem.USSR (Engl.Transl.); 38; (1968); 1645-1648; Tetrahedron Lett.; 27; 19; (1986); 2143-2144, Chem. Pharm. Bull.; EN; 17; 5; 1969; 873-878, Patent FR 1461184, Patent JP 13142.)

Die derzeitigen PET-Tracer, die für die Tumordiagnostik eingesetzt werden, haben einige unbestrittene Nachteile: So reichert sich FDG zwar bevorzugt in solchen Zellen mit erhöhtem Glukosestoffwechsel an, es gibt allerdings auch in anderen pathologischen und physiologischen Zuständen einen erhöhten Glukosestoffwechsel in den beteiligten Zellen und Geweben, z. Bsp. Infektionsherde oder Wundheilung (zusammengefasst in J. Nucl. Med. Technol. (2005), 33, 145-155). Es ist häufig immer noch schwierig zu entscheiden, ob eine mittels FDG-PET detektierte Läsion tatsächlich neoplastischen Ursprungs ist oder auf andere physiologische oder pathologische Zustände des Gewebes zurückzuführen ist. Insgesamt weist die Diagnosetätigkeit mittels FDG-PET in der Onkologie eine Sensitivität von 84% und eine Spezifität von 88% auf (Gambhir et al. "A tabulated summary of the FDG PET literature" J. Nucl. Med. 2001, 42, 1-93S). Tumore im Gehirn lassen sich beispielsweise durch die hohe Anreicherung von FDG in gesundem Hirngewebe nur sehr schwer darstellen.
Die bisher bekannten ¹⁸F markierten Aminosäurederivate sind in einigen Fällen gut geeignet, um Tumore im Gehirn zu detektieren ((review): Eur J Nucl Med Mol Imaging. 2002 May;29(5):681-90), allerdings können sie bei anderen Tumoren nicht mit den Bildgebungseigenschaften des "Goldstandards" [¹⁸F]2-FDG konkurrieren. Die metabolische Anreicherung und Retention der bislang F-18 markierten Aminosäuren in tumorösem Gewebe ist in der Regel niedriger als für FDG. Darüberhinaus, ist die Zugänglichkeit isomerenreiner F-18 markierter nichtaromatischer Aminosäuren chemisch höchst anspruchsvoll.
Ähnlich wie für Glukose wurde auch für Glutaminsäure und Glutamin ein erhöhter Metabolismus in proliferierenden Tumorzellen beschrieben (Medina, J Nutr. 1131:2539S-2542S, 2001; Souba, Ann Surg 218: 715-728, 1993). Die erhöhte Rate an Protein- und Nukleinsäuresynthesen sowie die Energiegewinnung per se werden als Gründe für einen verstärkten Glutaminkonsum von Tumorzellen angenommen. Die Synthese von entsprechenden C-11 und C-14 markierten, mit dem natürlichen Substrat also identischen Verbindungen, wurde in der Literatur bereits beschrieben (z. Bsp. Antoni, Enzyme Catalyzed Synthesis of L-[4-C-11]Aspartate and L-[5-C-11]Glutamate. J. Labelled Compd. Radiopharm. 44;( 4) 2001: 287 - 294) und Buchanan, The biosynthesis of showdomycin: studies with stable isotopes and the determination of principal precursors. J. Chem. Soc. Chem. Commun.; EN; 22; 1984; 1515-1517). Erste Hinweise mit der C-11 markierten Verbindung deuten auf keine signifikante Tumorakkumulation hin.

Aufgabe der vorliegenden Erfindung ist es neue Verbindungen zu finden, die sich in [¹⁸F] markierter Form für die PET-basierte Diagnose eignen.

Die Aufgabe wird gelöst durch die erfindungsgemäße Bereitstellung von [¹⁸F] markierte L-Glutaminsäure und [¹⁸F] markiertem L-Glutamin, sowie ihrer Derivate gemäß der allgemeinen Formel (I), einschließlich ihrer Diastereomere und Enantiomere: 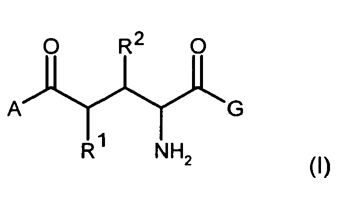 worin
A für
   a) Hydroxy
   b) verzweigtes oder unverzweigtes C₁-C₅ alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ alkyl)ₙ-O-C₁-C₄ alkyl
   e) N(C₁-C₅ Alkyl)₂,
   f) NH₂,
   g) N(H)-L,
   h) O-L, oder
   i) O-Z
   steht,
G für
   a) hydroxyl
   b) verzweigtes oder unverzweigtes O-C₁-C₅ Alkyl
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl oder
   e) verzweigtes oder unverzweigtes O-C₂-C₅ Alkinyl
   steht,
R¹ und R² für
   a) Wasserstoff
   b) ¹⁸_{F}
   c) verzweigtes oder unverzweigtes ¹⁸F C₁-C₅ Alkoxy,
   d) verzweigtes oder unverzweigtes ¹⁸F-C₁-C₅ Alkyl,
   e) verzweigtes oder unverzweigtes ¹⁸F-hydroxy-C₁-C₅ Alkyl,
   f) verzweigtes oder unverzweigtes ¹⁸F-C₂-C₅ Alkenyl, oder
   g) verzweigtes oder unverzweigtes ¹⁸F-C₂-C₅ Alkinyl,
   h) Hydroxyl
   i) verzweigtes oder unverzweigtes C₁-C₅ Alkyl oder
   j) verzweigtes oder unverzweigtes C₁-C₅ Alkoxy
   stehen, mit der Maßgabe, dass genau einer der Substituenten R¹ oder R² genau ein ¹⁸F Isotop beinhaltet und der jeweils andere Substituent kein ¹⁸F Isotop beinhaltet,
L für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl oder
   d) verzweigtes oder unverzweigtes C₂-C₅ Alkinyl
   steht, und
Z für ein Metallion steht,
wobei n = 0, 1, 2 oder 3 ist, und wobei alle möglichen Diastereomere und Enantiomere Teil des vorliegenden Erfindungsgegenstandes sind.

Bevorzugte Verbindungen der Erfindung gemäß der Formel (I) zeichnen sich dadurch aus, dass A für
a) Hydroxyl
b) Methoxy,
c) Ethoxy,
d) Propoxy,
e) NMe₂,
f) NEt₂
g) NH₂,
h) N(H)-L oder
i) O-L,
j) O-Z
steht.

Weitere bevorzugte Verbindungen der Erfindung gemäß der Formel (I) zeichnen sich dadurch aus,dass
A für
a) Hydroxyl,
b) Methoxy,
c) Ethoxy,
d) NMe₂,
e) NH₂, oder
f) NHL
steht.

Besonders bevorzugte Verbindungen der Erfindung gemäß der Formel (I) zeichnen sich dadurch aus, dass
A für
a) Hydroxyl,
b) Methoxy oder
c) NH₂
steht.

Bevorzugte Verbindungen der Erfindung gemäß der Formel (I) zeichnen sich dadurch aus, dass
A für
OH
steht.

Bevorzugte Verbindungen der Erfindung gemäß der Formel (I) zeichnen sich dadurch aus, dass
A für
NH₂
steht.

Bevorzugte Verbindungen der Erfindung gemäß der Formel (I) zeichnen sich dadurch aus, dass
G für
a) Hydroxyl
b) Methoxy,
c) Ethoxy,
d) Propoxy,
e) iso-Propoxy, oder
f) O-C₂H₄-OMe
steht.

Weitere bevorzugte Verbindungen der Erfindung gemäß der Formel (I) zeichnen sich dadurch aus, dass
G für
a) Hydroxyl
b) Methoxy, oder
c) Ethoxy
steht.

Besonders bevorzugte Verbindungen der Erfindung gemäß der Formel (I) zeichnen sich dadurch aus, dass
G für
a) Hydroxyl oder
b) Methoxy
steht.

Bevorzugte Verbindungen der Erfindung gemäß der Formel (I) zeichnen sich dadurch aus, dass
R¹ und R² für
a) Wasserstoff
b) ¹⁸F
c) ¹⁸F-methoxy,
d) ¹⁸F-ethoxy,
e) ¹⁸F-propoxy
f) ¹⁸F-methyl,
g) ¹⁸F-ethyl, oder
h) ¹⁸F-propyl
mit der Maßgabe, dass genau einer der Substituenten R¹ oder R² genau ein ¹⁸F-Isotop beinhaltet und der jeweils andere Substituent Wasserstoff ist,
steht.

Weitere bevorzugte Verbindungen der Erfindung gemäß der Formel (I) zeichnen sich dadurch aus, dass
R¹ und R² für
a) Wasserstoff
b) ¹⁸F
c) ¹⁸F-methoxy,
d) ¹⁸F-methyl, oder
e) ¹⁸F-ethyl,
mit der Maßgabe, dass genau einer der Substituenten R¹ oder R² genau ein ¹⁸F Isotop beinhaltet und der jeweils andere Substituent Wasserstoff ist,
steht.

Besonders bevorzugte Verbindungen der Erfindung gemäß der Formel (I) zeichnen sich dadurch aus, dass
R¹ und R² für
a) Wasserstoff
b) ¹⁸F oder
c) ¹⁸F-methyl
steht, mit der Maßgabe, dass genau einer der Substituenten R¹ oder R² genau ein ¹⁸F-Isotop beinhaltet und der jeweils andere Substituent Wasserstoff ist.

Besonders bevorzugte Verbindungen der Erfindung gemäß der Formel (I) zeichnen sich dadurch aus, dass R¹ ausgewählt ist aus der Gruppe OH, CH₃, C₂H₅, und C₃H₇ und R² für ¹⁸F steht.

Besonders bevorzugte Verbindungen der Erfindung gemäß der Formel (I) zeichnen sich dadurch aus, dass R² ausgewählt ist aus der Gruppe OH, CH₃, C₂H₅, und C₃H₇ und R¹ für ¹⁸F steht.

Besonders bevorzugte Verbindungen der Erfindung gemäß der Formel (I) zeichnen sich dadurch aus, dass R¹ für H und R² für ¹⁸F steht.

Besonders bevorzugte Verbindungen der Erfindung gemäß der Formel (I) zeichnen sich dadurch aus, dass R¹ für ¹⁸F und R² für H steht.

Bevorzugte Verbindungen der Erfindung gemäß der Formel (I) zeichnen sich dadurch aus, dass
L für
a) Methyl
b) Ethyl,
c) Propyl,
d) iso-Propyl,
e) -C₂H₄-OMe, oder
f) -C₂H₄-O-C₂H₄-OMe
steht.

Besonders bevorzugte Verbindungen der Erfindung gemäß der Formel (I) zeichnen sich dadurch aus, dass
L für
a) Methyl oder
b) Ethyl
steht.

Ebenfalls bevorzugte Verbindungen der Erfindung gemäß der Formel (I) zeichnen sich dadurch aus, dass
Z für Alkali- und Erdalkaliionen steht.

Alle möglichen Diastereomere und Enantiomere der bevorzugten Verbindungen nach Formel (I) sind Teil des vorliegenden Erfindungsgegenstandes.

Weiterhin besonders bevorzugt ist jede einzelne der Verbindungen aus der folgenden Gruppe, wobei alle möglichen Diastereomere und Enantiomere Teil des vorliegenden Erfindungsgegenstandes sind:

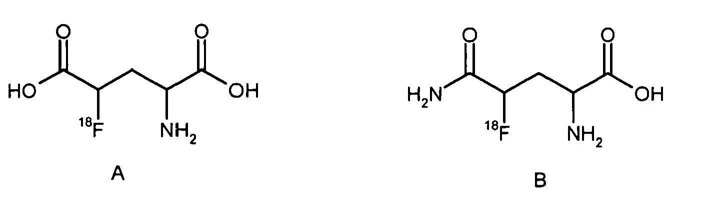

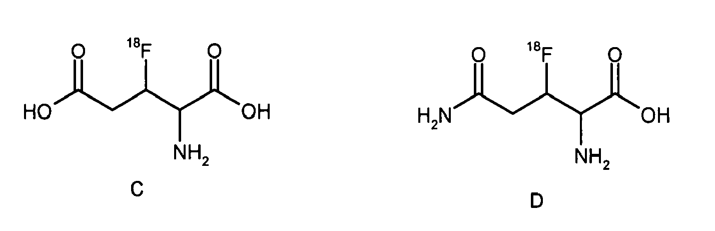

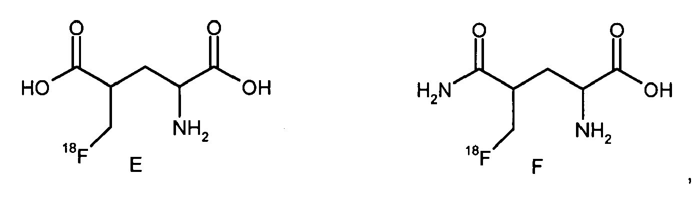

Das Verfahren zur Herstellung der erfindungsgemäßen Verbindungen der allgemeinen Formel (I) zeichnet sich dadurch aus, dass die Verbindung nach Formel (I), aus einer Vorläuferverbindung der Verbindung nach Formel (II) nach Einführung des ¹⁸F Isotops freigesetzt wird. In einem zweiten Aspekt bezieht sich somit die vorliegende Erfindung auf Verbindungen der Formel (II): 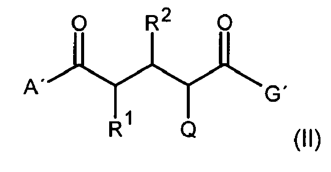
worin A' für
   a) Hydroxy
   b) verzweigtes oder unverzweigtes C₁-C₅ alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ alkyl)ₙ-O-C₁-C₄ alkyl
   e) N(C₁-C₅ Alkyl)₂,
   f) NH₂,
   g) N(H)-U
   h) N(H)-L', oder
   i) O-L'
   steht,
G'für
   a) hydroxyl
   b) O-Z'
   c) verzweigtes oder unverzweigtes O-C₁-C₅ Alkyl
   d) verzweigtes oder unverzweigtes O-C₂-C₅ Alkenyl,
   e) verzweigtes oder unverzweigtes O-C₁-C₅ Alkyl-(O-C₁-C₄ alkyl)ₙ-O-C₁-C₄ alkyl, oder
   f) verzweigtes oder unverzweigtes O-C₂-C₅ Alkinyl
   steht,
R¹ und R² für
   a Wasserstoff
   b) ¹⁸F
   c) verzweigtes oder unverzweigtes ¹⁸F C₁-C₅ Alkoxy
   d) verzweigtes oder unverzweigtes ¹⁸F-C₁-C₅ Alkyl,
   e) verzweigtes oder unverzweigtes ¹⁸F-C₁-C₅ Hydroxy-alkyl,
   f) verzweigtes oder unverzweigtes ¹⁸F-C₂-C₅ Alkenyl, oder
   g) verzweigtes oder unverzweigtes ¹⁸F-C₂-C₅ Alkinyl,
   h) Hydroxy
   i) verzweigtes oder unverzweigtes C₁-C₅ Alkyl, oder
   j) verzweigtes oder unverzweigtes C₁-C₅ Alkoxy
   steht, mit der Maßgabe, dass genau einer der Substituenten R¹ oder R² genau ein ¹⁸F Isotop beinhaltet und der jeweils andere Substituent kein ¹⁸F Isotop beinhaltet,
Q für
   a) N(H)-tert-Butoxycarbonyl,
   b) N(H)-Allyloxycarbonyl,
   c) N(H)-Benzyloxycarbonyl,
   d) N(H)-Ethoxycarbonyl
   e) N(H)-Methoxycarbonyl
   f) N(H)-Propoxycarbonyl
   e) N(H)-2,2,2-Trichlorethoxycarbonyl
   f) N(H)-1,1-Dimethylpropinyl
   g) N(H)-1-Methyl-1-phenyl-ethoxycarbonyl
   h) N(H)-1-Methyl-1-(4-biphenylyl)-ethoxycarbonyl
   i) N(H)-cyclobutylcarbonyl
   j) N(H)-1-Methylcyclobutylcarbonyl
   k) N(H)-Vinylcarbonyl
   l) N(H)-Allylcarbonyl
   m) N(H)-Adamantylcarbonyl
   n) N(H)-Diphenylmethylcarbonyl
   o) N(H)-Cinnamylcarbonyl
   p) N(H)-Formyl
   q) N(H)-Benzoyl
   r) N(H)-Trityl
   s) N(H)-p-Methoxyphenyl-diphenylmethyl
   t) N(H)-di-(p-Methoxyphenyl)-phenylmethyl, oder
   u) 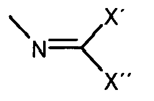
   v) N-(tert-Butoxycarbonyl)₂,
   steht,
L'für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl, oder
   d) verzweigtes oder unverzweigtes C₂-C₅ Alkinyl
   steht,
U für
   a) tert-Butoxycarbonyl,
   b) Allyloxycarbonyl,
   c) Benzyloxycarbonyl,
   d) Methoxycarbonyl,
   e) Propoxycarbonyl, oder
   d) Ethoxycarbonyl
   steht,
X und X' unanhängig voneinander für
   a) verzweigtes oder unverzweigtes C₁-C₅ Alkyl,
   b) substituiertes oder unsubstituiertes Aryl
   c) Aralkyl oder
   d) Heteroaryl
   und
Z' für ein Metallkation steht,
wobei n = 0, 1, 2 oder 3 ist und alle möglichen Diastereomere und Enantiomere Teil des vorliegenden Erfindungsgegenstandes sind.

Bevorzugte Verbindungen der Erfindung gemäß der Formel (II) zeichnen sich dadurch aus, dass
A'für
a) Hydroxyl
b) Methoxy,
c) Ethoxy,
d) tert-Butoxy,
e) NMe₂,
f) NEt₂
g) NH₂,
h) N(H)-U
i) N(H)-L' oder
j) O-L'
steht.

Weitere bevorzugte Verbindungen der Erfindung gemäß der Formel (II) zeichnen sich dadurch aus, dass
A' für
a) Hydroxyl,
b) Methoxy,
c) Ethoxy,
d) NMe₂,
e) N(H)-U
f) NH₂, oder
g) N(H)-L'
steht.

Besonders bevorzugte Verbindungen der Erfindung gemäß der Formel (II) zeichnen sich dadurch aus, dass
A' für
a) Hydroxyl,
b) Ethoxy
b) Methoxy,
c) N(H)-U oder
d) NH₂
steht.

Besonders bevorzugte Verbindungen der Erfindung gemäß der Formel (II) zeichnen sich dadurch aus, dass
A' für
a) OH,
b) verzweigtes oder unverzweigtes C₁-C₅ alkoxy,
c) -NH-tert-Butoxycarbonyl, oder
d) NH₂
steht.

Besonders bevorzugte Verbindungen der Erfindung gemäß der Formel (II) zeichnen sich dadurch aus, dass
A' für Ethoxy steht.

Besonders bevorzugte Verbindungen der Erfindung gemäß der Formel (II) zeichnen sich dadurch aus, dass
A' für NH₂ steht.

Bevorzugte Verbindungen der Erfindung gemäß der Formel (II) zeichnen sich dadurch aus, dass
G' für
a) Hydroxyl
b) OZ'
c) Methoxy,
d) Ethoxy,
e) tert-Butoxy
f) iso-Propoxy, oder
g) O-C₂H₄-OMe
steht.

Weitere bevorzugte Verbindungen der Erfindung gemäß der Formel (II) zeichnen sich dadurch aus, dass
G' für
a) Hydroxyl
b) OZ'
c) Methoxy, oder
d) Ethoxy
steht.

Besonders bevorzugte Verbindungen der Erfindung gemäß der Formel (II) zeichnen sich dadurch aus, dass
G' für
a) Hydroxyl
b) OZ'
c) Methoxy
steht.

Besonders bevorzugte Verbindungen der Erfindung gemäß der Formel (II) zeichnen sich dadurch aus, dass
G' für Ethoxy steht.

Bevorzugte Verbindungen der Erfindung gemäß der Formel (II) zeichnen sich dadurch aus, dass
R¹ und R² für
a) Wasserstoff
b) ¹⁸F
c) ¹⁸F-methoxy,
d) ¹⁸F-ethoxy,
e) ¹⁸F-propoxy
f) ¹⁸F-methyl,
g) ¹⁸F-ethyl, oder
h) ¹⁸F-propyl
steht, mit der Maßgabe, dass genau einer der Substituenten R¹ oder R² genau ein ¹⁸F-Isotop beinhaltet und der jeweils andere Substituent Wasserstoff ist.

Weitere bevorzugte Verbindungen der Erfindung gemäß der Formel (II) zeichnen sich dadurch aus, dass
R¹ und R² für
a) Wasserstoff
b) ¹⁸F
c) ¹⁸F-methoxy,
d) ¹⁸F-methyl, oder
e) ¹⁸F-ethyl,
steht, mit der Maßgabe, dass genau einer der Substituenten R¹ oder R² genau ein ¹⁸F-Isotop beinhaltet und der jeweils andere Substituent Wasserstoff ist.

Besonders bevorzugte Verbindungen der Erfindung gemäß der Formel (II) zeichnen sich dadurch aus, dass
R¹ und R² für
a) Wasserstoff
b) ¹⁸F oder
c) ¹⁸F-methyl,
steht, mit der Maßgabe, dass genau einer der Substituenten R¹ oder R² genau ein ¹⁸F-Isotop beinhaltet und der jeweils andere Substituent Wasserstoff ist.

Besonders bevorzugte Verbindungen der Erfindung gemäß der Formel (II) zeichnen sich dadurch aus, dass R¹ ausgewählt ist aus der Gruppe OH, CH₃, C₂H₅, und C₃H₇ und R² für ¹⁸F steht.

Besonders bevorzugte Verbindungen der Erfindung gemäß der Formel (II) zeichnen sich dadurch aus, dass R² ausgewählt ist aus der Gruppe OH, CH₃, C₂H₅, und C₃H₇ und R¹ für ¹⁸F steht.

Besonders bevorzugte Verbindungen der Erfindung gemäß der Formel (II) zeichnen sich dadurch aus, dass R¹ für H und R² für ¹⁸F steht.

Besonders bevorzugte Verbindungen der Erfindung gemäß der Formel (II) zeichnen sich dadurch aus, dass R¹ für ¹⁸F und R² für H steht.

Bevorzugte Verbindungen der Erfindung gemäß der Formel (II) zeichnen sich dadurch aus, dass
Q für
a) N(H)-tert-Butoxycarbonyl,
b) N(H)-Benzyloxycarbonyl,
c) N-(tert-Butoxycarbonyl)₂, oder
d) 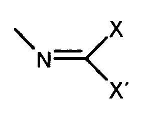
steht.

Weitere bevorzugte Verbindungen der Erfindung gemäß der Formel (II) zeichnen sich dadurch aus,dass
Q für
a) N(H)-tert-Butoxycarbonyl,
b) N(H)-Benzyloxycarbonyl,
c) N-(tert-Butoxycarbonyl)₂,
   oder
d) 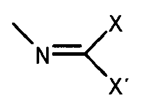
steht.

Besonders bevorzugte Verbindungen der Erfindung gemäß der Formel (II) zeichnen sich dadurch aus, dass
Q für
a) N(H)-Butoxycarbonyl, oder
b) 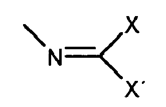
steht.

Besonders bevorzugte Verbindungen der Erfindung gemäß der Formel (II) zeichnen sich dadurch aus, dass
Q für N(H)-tert-Butoxycarbonyl steht.

Besonders bevorzugte Verbindungen der Erfindung gemäß der Formel (II) zeichnen sich dadurch aus, dass Q für 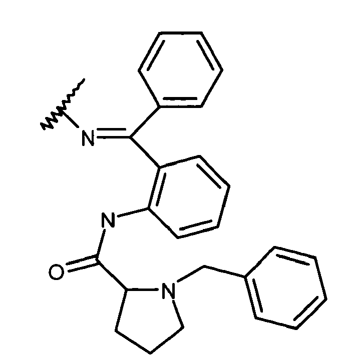 steht.

Bevorzugte Verbindungen der Erfindung gemäß der Formel (II) zeichnen sich dadurch aus, dass
L' für
a) Methyl
b) Ethyl,
c) Propyl,
d) iso-Propyl,
e) -C₂H₄-OMe, oder
f) -C₂H₄-O-C₂H₄-OMe
steht.

Weitere bevorzugte Verbindungen der Erfindung gemäß der Formel (II) zeichnen sich dadurch aus, dass
L' für
a) Methyl, oder
b) Ethyl
steht.

Bevorzugte Verbindungen der Erfindung gemäß der Formel (II) zeichnen sich dadurch aus, dass
U für
a) tert-Butoxycarbonyl,
b) Allyloxycarbonyl, oder
c) Ethoxycarbonyl steht.

Weitere bevorzugte Verbindungen der Erfindung gemäß der Formel (II) zeichnen sich dadurch aus,dass
U für
a) tert-Butoxycarbonyl, oder
b) Ethoxycarbonyl
steht.

Besonders bevorzugte Verbindungen der Erfindung gemäß der Formel (II) zeichnen sich dadurch aus, dass
U für
tert-Butoxycarbonyl
steht.

Bevorzugte Verbindungen der Erfindung gemäß der Formel (II) zeichnen sich dadurch aus, dass
X und X' unanhängig voneinander für
a) verzweigtes oder unverzweigtes C₁-C₅ Alkyl,
b) substituiertes oder unsubstituiertes Aryl oder
c) Alkylaryl
steht.

Weitere bevorzugte Verbindungen der Erfindung gemäß der Formel (II) zeichnen sich dadurch aus, dass
X und X' unabhängig voneinander für
a) verzweigtes oder unverzweigtes C₁-C₅ Alkyl, oder
b) Aryl
steht.

Besonders bevorzugte Verbindungen der Erfindung gemäß der Formel (II) zeichnen sich dadurch aus, dass
X und X' für Phenyl bzw. für in 2-Position substituiertes Phenyl
steht.

Bevorzugte Verbindungen der Erfindung gemäß der Formel (II) zeichnen sich dadurch aus, dass
Z' für Ni²⁺ Ion steht.

Wobei alle möglichen Diastereomere und Enantiomere der bevorzugten Verbindungen der Erfindung gemäß Formel (II) Teil des vorliegenden Erfindungsgegenstandes sind.

Weiterhin besonders bevorzugt ist jede einzelne der Verbindungen aus der folgenden Gruppe wobei alle möglichen Diastereomere und Enantiomere Teil des vorliegenden Erfindungsgegenstandes sind:

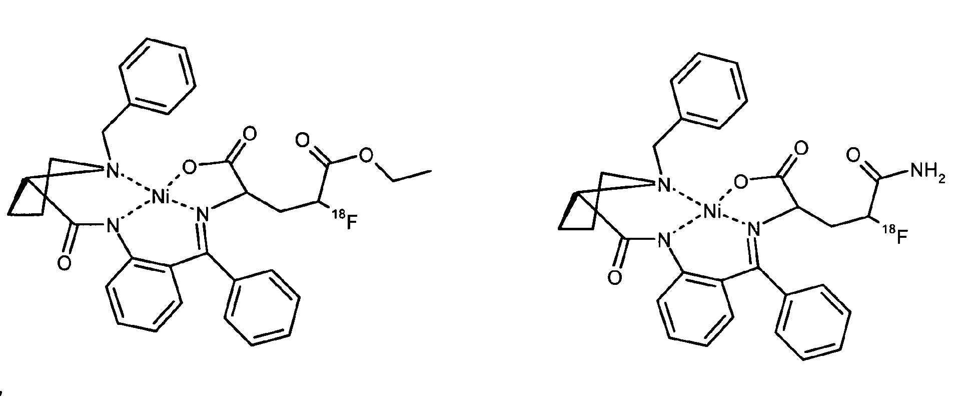

Das Verfahren zur Herstellung der erfindungsgemäßen Verbindungen der allgemeinen Formel (II) zeichnet sich dadurch aus, dass die Mehrzahl der Verbindung nach Formel (II), aus einer Vorläuferverbindung der Verbindung der Formel (III) nach Einführung des ¹⁸F Isotops entstehen kann.
In einem dritten Aspekt bezieht sich die vorliegende Erfindung auf Verbindungen der Formel (III): 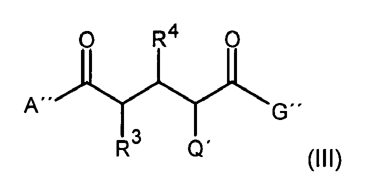 worin
A" für
   a) Hydroxy
   b) verzweigtes oder unverzweigtes C₁-C₅ alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ alkyl)ₙ-O-C₁-C₄ alkyl
   e) N(C₁-C₅ Alkyl)₂,
   f) NH₂,
   g) N(H)-U'
   h) N(H)-L" oder
   i) O-L"
   steht,
G" für
   a) hydroxyl
   b) O-Z"
   c) verzweigtes oder unverzweigtes O-C₁-C₅ Alkyl
   d) verzweigtes oder unverzweigtes O-C₂-C₅ Alkenyl,
   e) verzweigtes oder unverzweigtes O-C₁-C₅ Alkyl-(O-C₁-C₄ alkyl)ₙ-O-C₁-C₄ alkyl,
   f) verzweigtes oder unverzweigtes O-C₂-C₅ Alkinyl, oder
   g) triphenylmethoxy
   steht,
R³ und R⁴ für
   a) Wasserstoff
   b) verzweigtes oder unverzweigtes E-C₁-C₅ Alkoxy
   c) verzweigtes oder unverzweigtes E-C₁-C₅ Alkyl,
   d) verzweigtes oder unverzweigtes E-C₁-C₅ Hydroxy-alkyl,
   e) verzweigtes oder unverzweigtes E-C₂-C₅ Alkenyl,
   f) verzweigtes oder unverzweigtes E-C₂-C₅ Alkinyl,
   g) Hydroxy
   h) verzweigtes oder unverzweigtes C₁-C₅ Alkyl, oder
   i) verzweigtes oder unverzweigtes C₁-C₅ Alkoxy
   steht, mit der Maßgabe, dass genau einer der Substituenten R¹ oder R² ein E beinhaltet und der jeweils andere Substituent kein E beinhaltet,
E für
   a) Chloro,
   b) Bromo,
   c) Mesyloxy
   d) Trifluormesyloxy
   e) Nonafluorbutyloxy, oder
   f) Tosyloxy
   steht,
Q' für
   a) N(H)-tert-Butoxycarbonyl,
   b) N(H)-Allyloxycarbonyl,
   c) N(H)-Benzyloxycarbonyl,
   d) N(H)-Ethoxycarbonyl
   e) N(H)-Methoxycarbonyl
   f) N(H)-Propoxycarbonyl
   g) N(H)-2,2,2-Trichlorethoxycarbonyl
   h) N(H)-1,1-Dimethylpropinyl
   i) N(H)-1-Methyl-1-phenyl-ethoxycarbonyl
   j) N(H)-1-Methyl-1-(4-biphenylyl)-ethoxycarbonyl
   k) N(H)-Cyclobutylcarbonyl
   l) N(H)-1-Methylcyclobutylcarbonyl
   m) N(H)-Vinylcarbonyl
   n) N(H)-Allylcarbonyl
   o) N(H)-Adamantylcarbonyl
   p) N(H)-Diphenylmethylcarbonyl
   q) N(H)-Cinnamylcarbonyl
   r) N(H)-Formyl
   s) N(H)-Benzoyl
   t) N(H)-Trityl
   u) N(H)-p-Methoxyphenyl-diphenylmethyl
   v) N(H)-di-(p-Methoxyphenyl)-phenylmethyl, oder
   w) 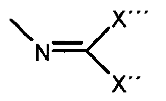
   x) N-(tert-Butoxycarbonyl)₂,
   steht,
L" für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl oder
   d) verzweigtes oder unverzweigtes C₂-C₅ Alkinyl
   steht,
U' für
   a) tert-Butoxycarbonyl,
   b) Allyloxycarbonyl,
   c) Benzyloxycarbonyl, oder
   d) Ethoxycarbonyl
   steht,
X" and X'" unanhängig voneinander für
   a) verzweigtes oder unverzweigtes C₁-C₅ Alkyl,
   b) substituiertes oder unsubstituiertes Aryl
   c) Alkylaryl oder
   d) Heteroaryl
   steht, und
Z" für Metallkationen steht,
wobei n = 0, 1 oder 2 ist und alle möglichen Diastereomere und Enantiomere Teil des vorliegenden Erfindungsgegenstandes sind.

Bevorzugte Verbindungen der Erfindung gemäß der Formel (III) zeichnen sich dadurch aus, dass
A" für
a) Hydroxyl
b) Methoxy,
c) Ethoxy,
d) tert-Butoxy,
e) NMe₂,
f) NEt₂
g) NH₂,
h) N(H)-U'
i) N(H)-L" oder
j) O-L"
steht.

Weiter bevorzugte Verbindungen der Erfindung gemäß der Formel (III) zeichnen sich dadurch aus, dass
A" für
a) Hydroxyl,
b) Methoxy,
c) Ethoxy,
d) NMe₂,
e) N(H)-U'
f) NH₂, oder
g) N(H)-L"
steht.

Besonders bevorzugte Verbindungen der Erfindung gemäß der Formel (III) zeichnen sich dadurch aus, dass
A" für
a) Hydroxyl,
b) Ethoxy
b) Methoxy,
c) N(H)-U, oder
d) NH₂
steht.

Bevorzugte Verbindungen der Erfindung gemäß der Formel (III) zeichnen sich dadurch aus, dass
G" für
a) Hydroxyl
b) OZ"
c) Methoxy,
d) Ethoxy,
e) tert-Butoxy
f) iso-Propoxy, oder
g) O-C₂H₄-OMe
steht.

Weiter bevorzugte Verbindungen der Erfindung gemäß der Formel (III) zeichnen sich dadurch aus, dass
G" für
a) Hydroxyl
b) OZ"
c) Methoxy, oder
d) Ethoxy
steht.

Besonders bevorzugte Verbindungen der Erfindung gemäß der Formel (III) zeichnen sich dadurch aus, dass
G" für
a) Hydroxyl
b) OZ"
c) Methoxy
steht.

Bevorzugte Verbindungen der Erfindung gemäß der Formel (III) zeichnen sich dadurch aus, dass
R³ und R⁴ für
a) Wasserstoff
b) E-methoxy,
c) E-ethoxy,
d) E-propoxy
e) E-methyl,
f) E-ethyl, oder
g) E-propyl
steht, mit der Maßgabe, dass genau einer der Substituenten R³ oder R⁴ ein E beinhaltet und der jeweils andere Substituent Wasserstoff ist.

Weiter bevorzugte Verbindungen der Erfindung gemäß der Formel (III) zeichnen sich dadurch aus, dass
R³ und R⁴ für
a) Wasserstoff
b) E-methoxy,
c) E-methyl, oder
d) E-ethyl
steht, mit der Maßgabe, dass genau einer der Substituenten R³ oder R⁴ ein E beinhaltet und der jeweils andere Substituent Wasserstoff ist.

Besonders bevorzugte Verbindungen der Erfindung gemäß der Formel (III) zeichnen sich dadurch aus, dass
R³ und R⁴ für
a) Wasserstoff oder
b) E-methyl
steht, mit der Maßgabe, dass genau einer der Substituenten R³ oder R⁴ ein E beinhaltet und der jeweils andere Substituent Wasserstoff ist.

Bevorzugte Verbindungen der Erfindung gemäß der Formel (III) zeichnen sich dadurch aus, dass
E für
a) Chloro,
b) Bromo,
c) Mesyloxy
d) Trifluormesyloxy oder
e) Tosyloxy
steht.

Weiter bevorzugte Verbindungen der Erfindung gemäß der Formel (III) zeichnen sich dadurch aus, dass
E für
a) Chloro,
b) Bromo,
c) Mesyloxy
d) Trifluormesyloxy oder
e) Tosyloxy
steht.

Besonders bevorzugte Verbindungen der Erfindung gemäß der Formel (III) zeichnen sich dadurch aus, dass
E für
a) Bromo, oder
b) Mesyloxy
steht.

Bevorzugte Verbindungen der Erfindung gemäß der Formel (III) zeichnen sich dadurch aus, dass
Q' für
a) N(H)-tert-Butoxycarbonyl,
b) N(H)-Benzyloxycarbonyl, oder
c) 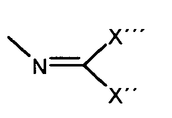
steht.

Weiter bevorzugte Verbindungen der Erfindung gemäß der Formel (III) zeichnen sich dadurch aus, dass
Q' für
a) N(H)-tert-Butoxycarbonyl, oder
b) 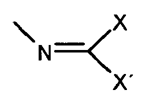
steht.

Bevorzugte Verbindungen der Erfindung gemäß der Formel (III) zeichnen sich dadurch aus, dass
L" für
a) Methyl
b) Ethyl,
c) Propyl,
d) iso-Propyl,
e) -C₂H₄-OMe, oder
f) -C₂H₄-O-C₂H₄-OMe
steht.

Weiter bevorzugte Verbindungen der Erfindung gemäß der Formel (III) zeichnen sich dadurch aus,dass
L" für
a) Methyl, oder
b) Ethyl
steht.

Bevorzugte Verbindungen der Erfindung gemäß der Formel (III) zeichnen sich dadurch aus, dass
U' für
a) tert-Butoxycarbonyl,
b) Allyloxycarbonyl, oder
c) Ethoxycarbonyl
steht.

Weiter bevorzugte Verbindungen der Erfindung gemäß der Formel (III) zeichnen sich dadurch aus, dass
U' für
a) tert-Butoxycarbonyl, oder
b) Benzyloxycarbonyl
steht.

Besonders bevorzugte Verbindungen der Erfindung gemäß der Formel (III) zeichnen sich dadurch aus, dass
U' für
tert-Butoxycarbonyl
steht.

Bevorzugte Verbindungen der Erfindung gemäß der Formel (III) zeichnen sich dadurch aus, dass
X" and X'" unanhängig voneinander für
a) verzweigtes oder unverzweigtes C₁-C₅ Alkyl,
b) substituiertes oder unsubstituiertes Aryl, oder
c) Alkylaryl
steht.

Weiter bevorzugte Verbindungen der Erfindung gemäß der Formel (III) zeichnen sich dadurch aus,dass
X" und X"' unabhängig voneinander für
a) verzweigtes oder unverzweigtes C₁-C₅ Alkyl, und
b) Aryl
steht.

Besonders bevorzugte Verbindungen der Erfindung gemäß der Formel (III) zeichnen sich dadurch aus, dass
X" und X'" für Phenyl bzw. für in 2-Position substituiertes Phenyl steht.

Bevorzugte Verbindungen der Erfindung gemäß der Formel (III) zeichnen sich dadurch aus, dass
Z" für Ni²⁺ steht.

Wobei alle möglichen Diastereomere und Enantiomere der bevorzugten Verbindungen gemäß Formel (III) Teil des vorliegenden Erfindungsgegenstandes sind.

Der Begriff "Aryl", so wie er hier selbst stehend oder als Teil einer anderen Gruppe angewendet wird, bezieht sich auf mono-zyklische oder zweifach-zyklische aromatische Gruppen, die sechs bis zwölf Kohlenstoffatome im Ring beinhalten können, wie z. Bsp. Phenyl oder Naphtyl, und in Position 2 beliebig substituiert sein können.

Die Erfindung umfasst auch den Ni^{II} Komplex enthaltend die Schiff'sche Base einer Verbindung nach Formel (I) und der Verbindung (S)-2-[N-(N-benzylprolyl)amino]benzophenon als solchen und seine Verwendung zur Herstellung einer Verbindung nach Formel (I).

Weiter umfasst die Erfindung auch den Ni^{II} Komplex enthaltend die Schiff'sche Base einer Vorläuferverbindung der Verbindung nach Formel (I) und der Verbindung (S)-2-[N-(N-benzylprolyl)amino]benzophenon als solchen und seine Verwendung zur Herstellung einer Verbindung nach Formel (I).

Erfindungsgemäße Verbindungen, wie z. B. 4-Fluoro-I-glutaminsäure und 4-Fluoro-l-glutamin lassen sich beispielsweise wie in Schema 1 dargestellt durch metallkatalysierte asymmetrische Synthese mit Hilfe eines Ni^{II}-Komplexes einer Glycin enthaltenden Schiff'schen Base und (S)-2-[*N*-(*N*-benzylprolyl)amino]benzophenon (BPB) (**1**)³ sowie Ethyl-α-Bromoacrylsäureethylester oder α-Bromoacrylamid (**2**) herstellen .

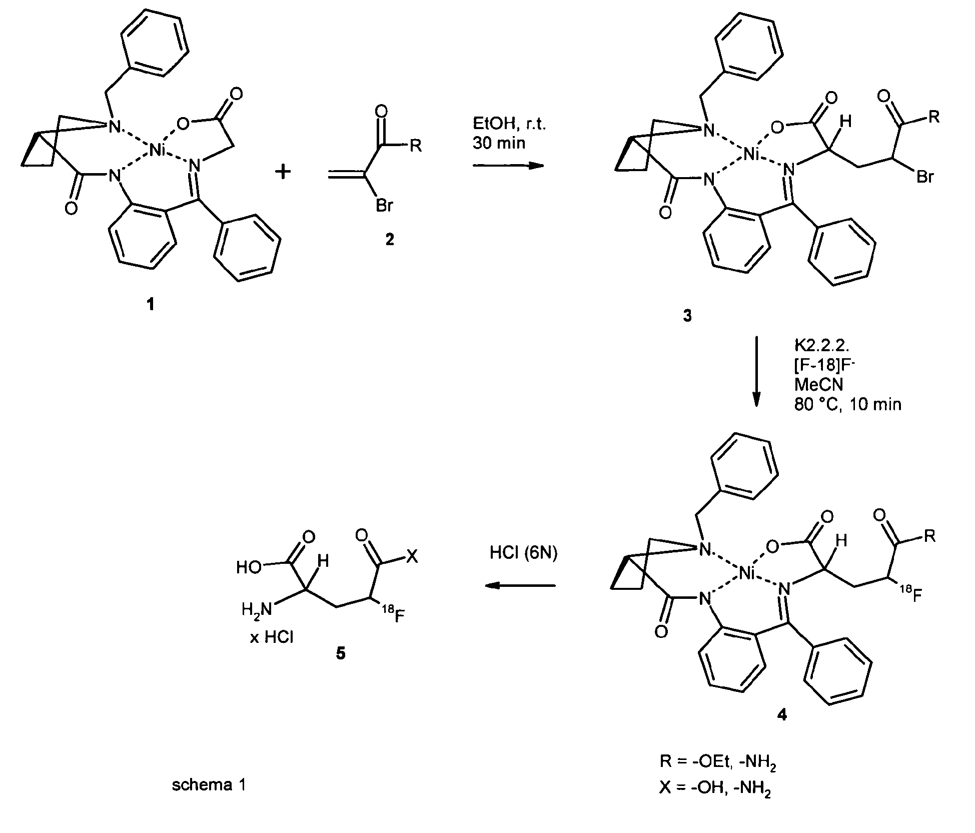

Dabei kann die C-C-Verknüpfung sowohl in protischen als auch aprotischen Lösungsmitteln erfolgen. Die Reaktion kann unter milden Bedingungen, wie zum Beispiel Raumtemperatur, oder erhöhten Temperaturen durchgeführt werden.
Dabei ist der Zusatz einer Base hilfreich. So kann zum Beispiel Diisopropylamin, Diisopropylethylamin, Triethylamin oder ähnliches verwendet werden. Die Reaktionsmischung wird günstiger Weise für 1 - 3 h gerührt und anschließend erneut mit Ethyl-α-bromoacrylat oder α-Bromoacrylamid versetzt. Die Reaktion kann mittels DC verfolgt werden. Hierzu eignen sich unter anderem Kieselgel/ Essigester/Chloroform-Systeme. Nach der Zugabe von Ethyl-α-Bromoacrylat oder α-Bromoacrylamid und mindestens 1 stündigem Rühren, ist die Reaktion zu einem erheblichen Teil abgeschlossen. Die Reaktionsmischung wird dann neutralisiert durch die Zugabe von organischer oder mineralischer Säure (oder einer Kombination aus beidem), zum Beispiel Essigsäure, Ameisensäure, Trifluoressigsäure, Trichloressigsäure, Propionsäure, Salzsäure, Schwefelsäure, Perchlorsäure, Phosphorsäure etc. Der 4-Bromo-glutaminsäureethylester-Ni-Komplex kann extraktiv aus dem Reaktionsgemisch separiert werden. Dafür eignen sich zum Beispiel organische halogenierte oder nicht-halogenierte Lösungsmittel, wie z.
Bsp. Chloroform, Methylenchlorid, Dialkylether, Essigester, Alkane, etc. Nach Trocknung der organischen Phase mittels Trockenmittel (z. Bsp. Natriumsulfat, Calziumsulfat oder ähnlichem) wird im Vakuum bis zur Trockene eingeengt.
Durch präparative DC (z. Bsp. an Kieselgel mit einem Laufmittelgemisch aus AcOEt/ CHCl₃, **1**: 1) kann eine Mischung stereoisomerer Komplexe wie **3** (S,S,S) und **3** (S,R,S/R) festgestellt werden. Komplex **3** (S,S,R) kann zum Beispiel in vorbenanntem Trennsystem mit einen *R*_{f}-Wert von 0.49 separiert werden. Die Komplexe **3** können z. Bsp. mit MeOH, EtOH etc. vom Kieselgel eluiert und anschließend durch Säulenchromatographie, z. Bsp. an Sephadex mit EtOH/ C₆H₆ Mischungen gereinigt werden.
Der erhaltene Komplex kann dann für weitere Substitutionsreaktionen an dem mit Brom substituierten Kohlenstoffatom eingesetzt werden.
Die erfindungsgemäße Vorläuferverbindung **3** kann durch nukleophile Substitution mit [¹⁸F]F⁻ in die entsprechende fluorierte Vorläuferverbindung **4** überführt werden. Dazu können die Komplexe **3** in Gegenwart einer Base wie zum Beispiel NBu₄OH, (NBu₄)₂CO₃, K₂CO₃ etc. mit der entsprechenden Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beinflussen, besonders in Kombination mit K₂CO₃ als katalysierende Base.
Die fluorierten Verbindungen 4-Fluoroglutaminsäure oder 4-Fluoroglutamin können durch Behandlung mit Säuren, wie z. Bsp. Salzsäure, Phosphorsäure, Perchlorsäure, Schwefelsäure etc. aus den entsprechenden Komplexen **3** freigesetzt werden. Dabei kommt es sowohl zur Abspaltung des Aminosäurederivates als auch zur Spaltung des Esters in 5-Position bei der Verwendung von 4-Fluoroglutaminsäure-5-methylester.
4-Fluoroglutaminsäure kann über eine Kartusche (z. Bsp. QMA (Waters), LiChrolut (VWR/Merck) or WHAT6803-2005 SPE COLSAX (VWR/ Merck)) vorgereinigt werden. Die Abtrennung von Verunreinigungen (Komplexverbindung **3**, **4**, **4-**Bromoglutaminsäure etc.) kann mittels HPLC erfolgen. Geeignete HPLC-Systeme können z. Bsp. sein: Säule: aminogruppentragendes Kieselgel (z. Bsp. Zorbax-NH₂); Eluent: 20 mM NaH₂PO₄ in Wasser, Fluß: 4 ml/ min Die gereinigte Verbindung **5** kann durch geeignetes Entfernen der HPLC-Lösungsmittel konzentriert und für eine Verwendung im Sinne dieser Erfindung eingesetzt werden. Die Entfernung der HPLC-Lösungsmittel kann auf unterschiedliche Weise erfolgen. Geeignet ist ein Einengen am Rotationsverdampfer unter vermindertem Druck, eine Aufheizung der Probe im Heizblock unter Stickstoffstrom etc. oder die Aufbringung auf eine Konzentratorkartusche (z. Bsp. C-18 SepPack etc.), gefolgt von einer Elution mit wenig EtOH/ wässriger Kochsalzlösung oder ähnlichem mit anschließender weiterer Konzentration durch vorgenannte Methoden.
Erfindungsgemäße Verbindungen, bei denen das ¹⁸F-Isotop in β-Stellung positioniert ist, wie z. Bsp. bei (2S)-3-Fluoro-glutaminsäure (**6**), lassen sich wie in Schema 2 dargestellt herstellen. So gelingt beispielshalber die saure Abspaltung der Schutzgruppen der Verbindung 7**7**, um die erfindungsgemäße Verbindung (2S)-3-Fluoro-glutaminsäure (**6**) zu erhalten. 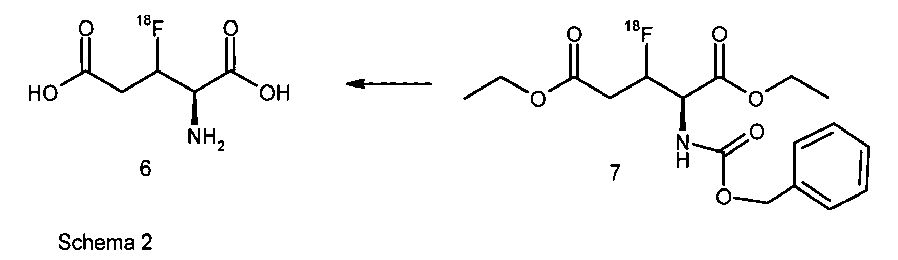 Dabei können verschiedene organische, vor allem aber anorganische Säuren wie z. Bsp. Bromwasserstoffsäure, Salzsäure, Schwefelsäure, Perchlorsäure oder Phosphorsäure zum Einsatz kommen. Eine Abspaltung der Schutzgruppen unter stark basischen Bedingungen mit z. Bsp. Natriumhydroxydlösung oder Kaliumhydroxydlösung ist weniger günstig, prinzipiell aber auch anwendbar und durchführbar. Die Reinigung der erfindungsgemäßen Verbindung **6** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **8**, dessen Synthese in der Literatur beschrieben ist (Chem. Pharm. Bull., 17, 5, (1969), 879-885), zum ¹⁸F-markierten Glutaminsäurederivat **7** ist nach den dem Fachmann bekannten Methoden durchführbar (s. Schema 3). 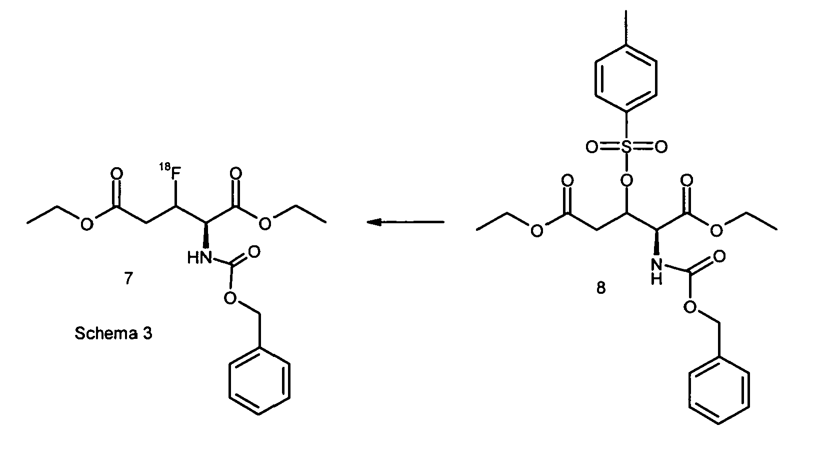 Dabei kann Verbindung **8** in Gegenwart einer Base wie zum Beispiel Tetra-alkyl ammonium und Tetra-alkl-phosphonium-carbonat und Kaliumcarbonat etc. mit der entsprechenden [¹⁸F]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [¹⁸F]-Fluoridanionen Acetonitril, Dimethylsulfoxid oder Dimethylformamid als optimale Lösungsmittel angewandt. Verbindung **7** muss üblicherweise keiner Reinigung unterzogen werden, sondern kann umgehend mit den für die Umsetzung von **7** nach **6** beschriebenen Methoden behandelt werden. Eine Reinigung der Verbindung **7** ist aber prinzipiell möglich, bevorzugt mittels einer präparativen HPLC mit einer unpolaren Phase, wie z. Bsp. RP C-18.

Erfindungsgemäße Verbindungen, bei denen das ¹⁸F-Isotop über eine Methylengruppe in 4-Position des Glutaminsäuregerüstes positioniert ist, wie z. Bsp. bei [¹⁸F](2S)-4-Fluoromethylglutaminsäure (**9**), lassen sich wie in Schema 4 dargestellt, herstellen. So gelingt beispielshalber die saure Abspaltung der Schutzgruppen der Verbindung **10**, um die erfindungsgemäße Verbindung (2S)-4-Fluoromethyl-glutaminsäure (**9**) zu erhalten. 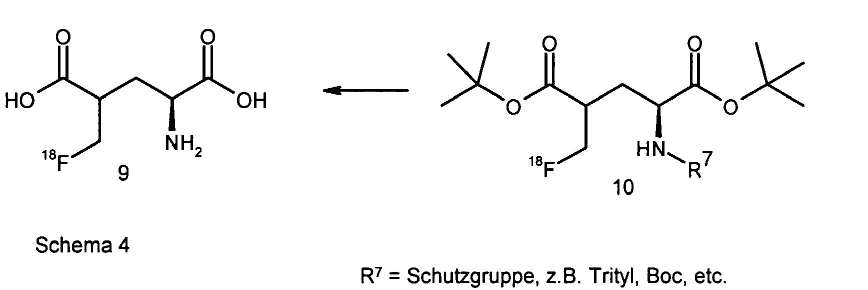 Dabei können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Die Reinigung der erfindungsgemäßen Verbindung **9** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **11**, dessen Synthese analog der in der Literatur beschriebenen Methode (Chem. Pharm. Bull., 17, 5, (1969), 879-885) aus **12** (Tetrahedron, 45, 5, (1989) 1453-1464) erfolgte, zum ¹⁸F-markierten Glutaminsäurederivat **10** ist nach den dem Fachmann bekannten Methoden durchführbar (s. Schema 5).

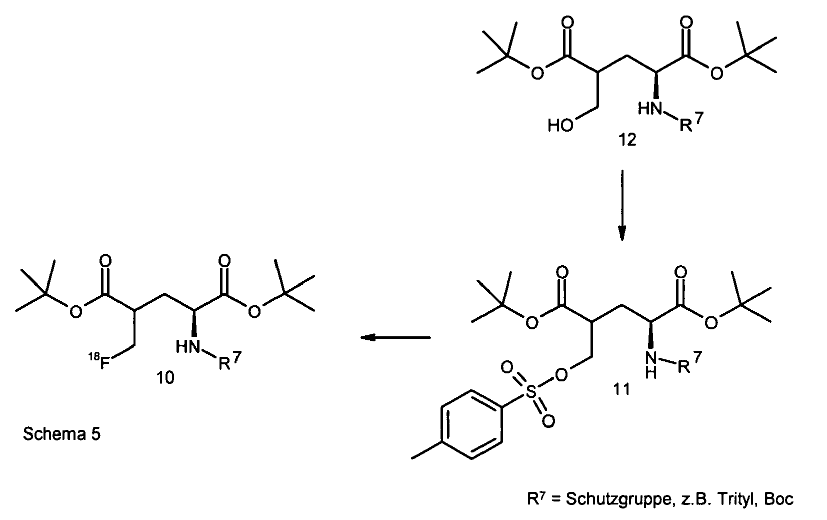

Dabei kann Verbindung **11** in Gegenwart einer Base wie zum Beispiel Tetra-alkyl ammonium und Tetra-alkyl-phosphonium-carbonat und Kaliumcarbonat etc. mit der entsprechenden [¹⁸F]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [¹⁸F]-Fluoridanionen Acetonitril, Dimethylsulfoxid oder Dimethylformamid als optimale Lösungsmittel angewandt. Verbindung **10** muss üblicherweise keiner Reinigung unterzogen werden, sondern kann umgehend mit den für die Umsetzung von **10** nach **9** beschriebenen Methoden behandelt werden. Eine Reinigung der Verbindung **10** ist aber prinzipiell möglich, bevorzugt mittels einer präparativen HPLC mit einer unpolaren Phase, wie z. Bsp. RP C-18.

In einem vierten Aspekt der Erfindung werden Verbindungen der Formel (IV) verwendet zur Herstellung von Verbindungen der Formel (I) oder (II): 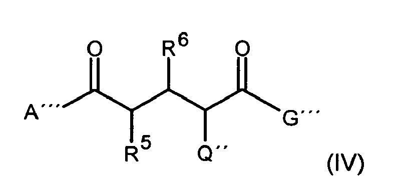 worin
A'" für
   a) hydroxy
   b) verzweigtes oder unverzweigtes C₁-C₅ alkoxy,
   c) verzweigtes oder unverzweigtes Hydroxy C₁-C₅ Alkoxy,
   d) verzweigtes oder unverzweigtes O-C₁-C₅ Alkyl-(O-C₁-C₄ alkyl)ₙ-O-C₁-C₄ alkyl
   e) N(C₁-C₅ Alkyl)₂,
   f) NH₂,
   g) N(H)-U"
   h) N(H)-L'" oder
   i) O-L"'
   steht,
G"' für
   a) hydroxyl
   b) O-Z"
   c) verzweigtes oder unverzweigtes O-C₁-C₅ Alkyl
   d) verzweigtes oder unverzweigtes O-C₂-C₅ Alkenyl,
   e) verzweigtes oder unverzweigtes O-C₁-C₅ Alkyl-(O-C₁-C₄ alkyl)ₙ-O-C₁-C₄ alkyl oder
   f) verzweigtes oder unverzweigtes O-C₂-C₅ Alkinyl,
   g) triphenylmethoxy
   steht,
R⁵ und R⁶ für
   a) Wasserstoff oder
   b) E'
   steht, mit der Maßgabe, dass genau einer der Substituenten R⁵ oder R⁶ ein E beinhaltet und der jeweils andere Substituent kein E beinhaltet,
E' für
   a) Chloro,
   b) Bromo,
   c) Mesyloxy
   d) Trifluormesyloxy
   e) Nonafluorbutyloxy oder
   f) Tosyloxy
   steht,
Q" für
   a) N(H)-tert-Butoxycarbonyl,
   b) N(H)-Allyloxycarbonyl,
   c) N(H)-Benzyloxycarbonyl,
   d) N(H)-Ethoxycarbonyl
   e) N(H)-Methoxycarbonyl
   f) N(H)-Propoxycarbonyl
   g) N(H)-2,2,2-Trichlorethoxycarbonyl
   h) N(H)-1,1-Dimethylpropinyl
   i) N(H)-1-Methyl-1-phenyl-ethoxycarbonyl
   j) N(H)-1-Methyl-1-(4-biphenylyl)-ethoxycarbonyl
   k) N(H)-cyclobutylcarbonyl
   l) N(H)-1-Methylcyclobutylcarbonyl
   m) N(H)-Vinylcarbonyl
   n) N(H)-Allylcarbonyl
   o) N(H)-Adamantylcarbonyl
   p) N(H)-Diphenylmethylcarbonyl
   q) N(H)-Cinnamylcarbonyl
   r) N(H)-Formyl
   s) N(H)-Benzoyl
   t) N(H)-Trityl
   u) N(H)-p-Methoxyphenyl-diphenylmethyl
   v) N(H)-di-(p-Methoxyphenyl)-phenylmethyl, oder
   w) 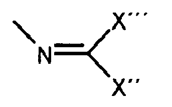
   x) N-(tert-Butoxycarbonyl)₂,
   steht,
L"' für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄ alkyl, oder
   d) verzweigtes oder unverzweigtes C₂-C₅ Alkinyl
   steht,
U" für
   a) tert-Butoxycarbonyl,
   b) Allyloxycarbonyl,
   c) Benzyloxycarbonyl, oder
   d) Ethoxycarbonyl
   steht,
X" and X"' unanhängig voneinander für
   a) verzweigtes oder unverzweigtes C₁-C₅ Alkyl,
   b) substituiertes oder unsubstituiertes Aryl
   c) Alkylaryl, oder
   d) Heteroaryl
   steht, und
Z" für Metallkationen steht,
wobei n = 0, 1, 2 oder 3 ist und alle möglichen Diastereomere und Enantiomere Teil des vorliegenden Erfindungsgegenstandes sind.

Wenn ein oder mehrere chirale Zentren in einer Verbindung des vorliegenden Erfindungsgegenstandes der Formel (I), Formel (II), Formel (III) oder (IV) vorhanden ist bzw. sind, so sollen alle Formen dieses Isomers, einschließlich beider Enantiomere und aller möglichen Diastereomere, hierin beinhaltet sein. Verbindungen, die mindestens ein chirales Zentrum enthalten, können als racemische Mischung, gegebenenfalls als Diastereomeren- oder Diastereomeren-angereicherte Mischung oder als Enantiomeren-angereicherte Mischung eingesetzt werden. Die racemische, enantiomeren-angereicherte Mischung oder Diastereomerenmischung kann gegebenenfalls nach den dem Fachmann bekannten Methoden getrennt werden, so dass die Enantiomere oder Diastereomere einzeln eingesetzt werden können. In solchen Fällen, in denen eine Kohlenstoff-Kohlenstoff-Doppelbindung vorliegt, so sind beide Isomere "cis" und "trans" Teil der vorliegenden Erfindung. In solchen Fällen, in denen tautomere Formen vorliegen können, wie z. Bsp. Keto-enol Tautomerie, sind alle tautomeren Formen in der vorliegenden Erfindung beinhaltet, wobei diese Formen im Gleichgewicht oder bevorzugt in einer Form vorliegen können.

Die Verbindungen der allgemeinen Formel I und ihre bevorzugten Ausführungsformen werden verwendet als Arzneimittel.
Die erfindungsgemäßen Verbindungen der allgemeinen Formel I und ihre bevorzugten Ausführungsformen werden verwendet in der Diagnose von physiologischen oder pathologischen Zuständen.
Bevorzugt finden diese Verbindungen Anwendung in der nicht-invasiven PET-basierten Diagnose am menschlichen oder tierischen Körper.

Besonders bevorzugt finden die erfindungsgemäßen Verbindungen der allgemeinen Formel I und ihre bevorzugten Ausführungsformen Anwendung in der Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich hormonabhängigem und hormonunabhängigem Brustkrebs, Plattenepithelkarzinom, neurologische Krebserkrankungen einschließlich Neuroblastom, Gliom, Astrocytom, Osteosarcom, Meningiom; Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.
Die erfindungsgemäßen Verbindungen der allgemeinen Formel I und ihre bevorzugten Ausführungsformen werden verwendet zur Herstellung eines Arzneimittels für die Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich Hormon-abhängigem und Hormon-unabhängigem Brustkrebs, Plattenepithelkarzinom, neurologische Krebserkrankungen einschließlich Neuroblastom, Gliom, Astrocytom, Osteosarcom, Meningiom, Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

### Beispiele:

### Beispiel 1:

### Synthese von [F-18]4-Fluoroglutaminsäure

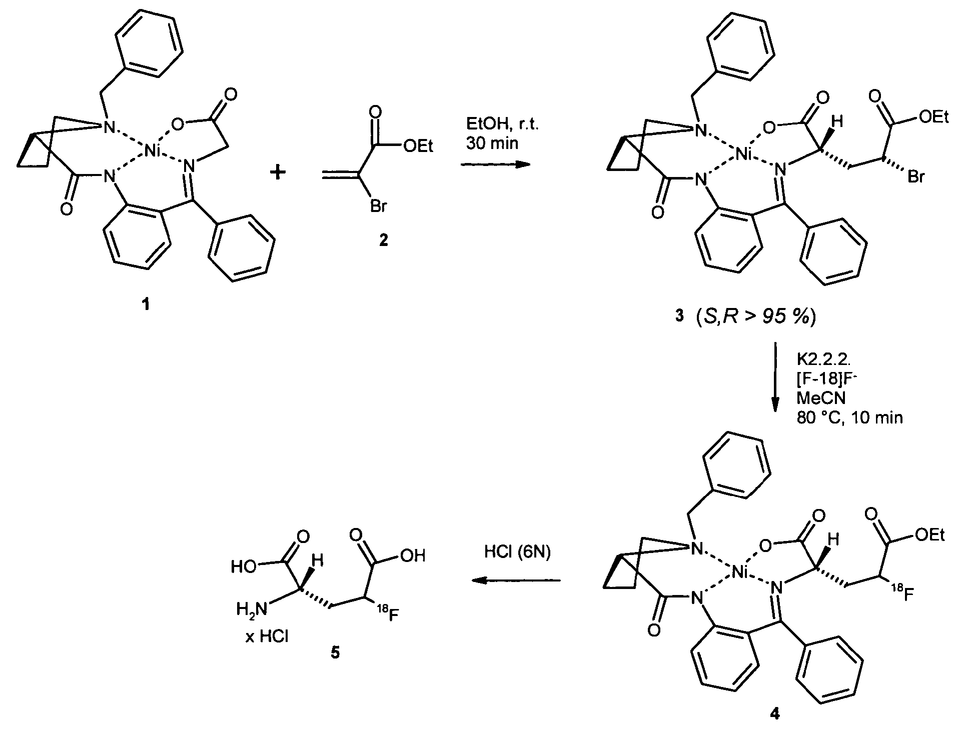

### Synthese von 3:

Diisopropylamin (0.031 ml, 0.22 mmol) wurde zu einer Suspension von Komplex **1** (0.1 g, 0.2 mmol) (J. Am. Chem. Soc., 1985, 107, 4252 oder Tetrahedron Asymmetry, 1998, 9, 4249) in EtOH (0.4 ml) bei Raumtemperatur (RT) gegeben. Die Reaktionsmischung wurde für 30 min. gerührt und anschließend mit frisch destilliertem Ester **2** (0.04 mL, 0.33 mmol) (Gazz. Chim. Ital., 1981, 111, 249) versetzt. Die Reaktion wurde mittels DC verfolgt (SiO₂, AcOEt/ CHCl₃, 1 : 1). Nach Abschluß der Reaktion (~ 2.5 h) wurde die Reaktionsmischung neutralisiert durch die Zugabe von AcOH (1.5 ml; 2 %). Danach wurde CHCl₃ (15 ml) zugegeben, mit Wasser gewaschen (3 x 15 ml), die organische Phase separiert, über Na₂SO₄ getrocknet und im Vakuum bis zur Trockene eingeengt. Preparative DC (SiO₂, AcOEt/ CHCl₃, 1 : 1) lieferte eine Mischung der Komplexe **3** (S,S,S) und **3** (S,R,S/R) in einem Verhältnis von 11 : 1 und einer Ausbeute von 28% (*R*_{f} 0.52). Komplex **3** (S,S,R) zeigte einen *R*_{f}-Wert von 0.49. Komplex **3** (S,S,R) wurde mit MeOH (3 x 40 mL) vom Kieselgel eluiert und anschließend durch Säulenchromatographie an Sephadex mit einer 1 : 3 EtOH/ C₆H₆ Mischung gereinigt. Die Reinigung lieferte 0.052 g (52%) of des Produktes. Elementaranalyse: gefunden (%): C, 56.79; H, 4.85; Br, 12.14; N, 6.10; Ni, 8.17. C₃₂H₃₂BrN₃NiO₅. Kalkuliert (%) C, 56.75; H, 4.76; Br, 11.80; N, 6.20; Ni, 8.67.

### F-19 L-Glutaminsäure (Nichttradioaktiver Standard für HPLC Identifikation von F-18 L-Glutaminsäure)

**Kondensation des Ni-BPB-Gly mit 2-Fluoro-acrylsäuremethylether.** Zu einer Suspension von 3 g (6 mmol) Ni-BPB-Gly in 15 ml MeOH wurde 1 ml (7.2 mmol) i-Pr₂NH bei RT gegeben. Die Reaktionsmischung wurde 30 min. gerührt und anschließend mit 3.7 ml (30 mmol) 2-Fluoro-acrylsäuremethylether versetzt. Der Fortschritt der Reaktion wurde mittels DC an SiO₂ (AcOEt:/CHCl₃ (2:3)) verfolgt. Nach ca. 250 Stunden war die Reaktion abgeschlossen. Danach wurde die Mischung neutralisiert durch Zugabe von 42 ml einer 2 % igen wässrigen Lösung von AcOH gemischt mit 25 ml MeOH. Die resultierende Mischung der diasteroisomeren Komplexe Ni-BPB-4-F-GluOMe wurde ausgefällt. Der Niederschlag wurde abfiltriert und mit Wasser (3 × 30 ml) gewaschen. Der resultierende feste Komplex wurde in CCl₄ suspendiert und im Vakuum zur Trockene eingeengt. Diese Prozedur wurde drei mal wiederholt um die Mischung von Wasser zu befreien. Die Komplexe wurden durch Säulenchromatographie (SiO₂, 3 ×20 cm, AcOEt/CHCl₃, 3:2) gereinigt. Die Hauptfraktion, 2.66 g, (4.4 mmol, 74 %) enthielt eine Mischung von Ni-BPB-(2S,4R)-4-F-GluOMe und Ni-BPB-(2S,4S)-4-F-GluOMe in einem Verhältnis von 1,5/ 1. Schmelzpunkt: 191 - 193 °C. [α]D25 +2477 (c 0.5, CHCl3). Elementaranalyse: gefunden (%): C, 61,76; H, 5,02; N, 6,94; Ni 9,20. Berechnet für C₃₁H₃₀FN₃NiO₅ (%) C, 61,82; H, 5,02; N, 6,98; Ni, 9,74.

**Separierung der Komplexe** wurde durch säulenchromatographische Trennung an Toyopearl HW-55F (Säule 2 × 50 cm, THF/ C₆H₆ (2 : 7)) erzielt.
Komplex Ni-BPB-(2S,4R)-4-F-GluOMe: Schmelzpunkt: 207 - 208 °C., [α]_{D}²⁵ +2617 (c 0.035, MeOH). Elementaranalyse: gefunden (%): C, 61,79; H, 4,95; N, 6,88. Berechnet für C₃₁H₃₀FN₃NiO₅ (%) C, 61,82; H, 5,02; N, 6,98.
Komplex Ni-BPB-(2S,4S)-4-F-GluOMe. Schmelzpunkt: 233 - 235 °C., [α]_{D}²⁵ +2641 (c 0,039, MeOH). Elementaranalyse: gefunden (%): C, 61,63; H, 4,86; N, 6,84. Berechnet für C₃₁H₃₀FN₃NiO₅ (%) C, 61,82; H, 5,02; N, 6,98.
***Zersetzung des Komplexes und Isolierung der Aminosäure.*** In einem Rundkolben wurden 2.75 g (4.57 mmol) Ni-BPB-4-F-GluOMe-Komplex in 30 ml MeOH gelöst und unter Rühren mit 5.5 ml HCl (6N) versetzt. Die Reaktionsmischung wurde für 15-20 min. am Rückfluß erhitzt, zur Trockene eingeengt, mit 50 ml Wasser verdünnt, das Hydrochlorid des BPB abfiltriert und vorsichtig mit 3 × 30 ml Wasser gewaschen. Die vereinigten Filtrate enthalten die Aminosäure, BPB-Rückstände und Ni²⁺-Salze und wurden mit wässriger NH₃-Lösung auf pH 5 eingestellt. BPB-Rückstände wurden mit CHCl₃ (3 × 30 ml) extraktiv entfernt. Die vereinigten wässrigen Phasen wurden zur Trockene eingeengt, mit 3 ml HCl (6N) versetzt und für 1 h am Rückfluß erhitzt. Die Lösung wurde anschließend zur Trockene eingeengt, in 5 ml H₂O gelöst und mit 5 % iger (wässrig) NH₃-Lösung auf pH 4 eingestellt. Die Aminosäure wurde mittels Ioneneaustauschchromatographie über eine Dowex-Säule 50w×8 in H⁺-Form (Eluent: 5% NH₃ aq) isoliert.
(2*S*,4*R*)-4-Fluoroglutaminsäure: Schmelzpunkt: >300 °C (Zersetzung ohne Schmelzpunkt).
(2*S*,4*S*)-4-Fluoroglutaminsäure: Schmelzpunkt: >300 °C (Zersetzung ohne Schmelzpunkt).

### F-18 Radiomarkierung:

Target: Kleinvolumiges Niederdruck-Silber-Target (1 ml) gefüllt mit [O-18] Wasser für ¹⁸O (p,n)¹⁸F Reaktion; Zyklotron: Scanditronix MC 17; Protonenbeschuß bei 17 MeV.
F-18 Fluorid wurde auf einer QMA-Harzkartusche (Waters, Sep Pak Light QMA Part.No.: WAT023525) durch Aufgabe der [F-18]/[O-18] Targetlösung konzentriert. Die Kartusche wurde mit K₂CO₃-Lösung (10 ml; 0.5 M) gefolgt von deionisiertem Wasser (15 ml) präkonditioniert.

[F-18] radioaktiver Komplex: [F-18]Fluorid (15 - 300 mCi) wurde mittels Waschlösung (2 ml, MeCN (2 ml)/ Tetrabutylammoniumkarbonat (TBAC, 0,015 mL, 20 % wässrig, pH 8)) von der QMA-Kartusche eluiert. Das Eluat wurde in einem 5 ml Vial aufgefangen und die Lösungsmittel durch Azeotropdestillation bei 130°C im Stickstoffstrom entfernt.
Nucleophile Substitution: Das Reaktionsgefäß mit dem trockenen [F-18] TBA-fluorid wurde auf 80 °C (vorhergehender Schritt) abgekühlt und eine Lösung von Präkursor 3 (S,S,R) (5 mg in MeCN (0.5 ml)) wurde zugesetzt. Die Reaktionsmischung wurde für 5 - 10 min. bei 80 °C gehalten. Eine Probe der Reaktionsmischung wurde mittels Radio-DC (Kieselgelplatte (Merck), Eluent: Ethylacetate/ Chloroform/ Essigsäure (4/1/1) untersucht. Basierend auf diesen Radio-DC-Daten wurde eine F-18-Inkorporation von 40 - 60 % in 4 ermittelt werden.

Zersetzung des ¹⁸F-fluorierten Ni-Komplexes und Freisetzung von 4-[¹⁸F]Fluoroglutamisäure (A). HCl (6 N, 0.3 - 0.5mL) wurde zur MeCN-Lösung des F-18-Glutamate-Nickelpräkursors gegeben und bei 140 °C für 5 min. behandelt. Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.
Zersetzung des ¹⁸F-fluorierten Ni-Komplexes und Freisetzung von 4-[¹⁸F]Fluoroglutamisäure (B). HCl (2 N, 0.3 - 0.5mL) wurde zur MeCN-Lösung des F-18-Glutamate-Nickelpräkursors gegeben und bei 140 °C für 5 min. behandelt. Anschließend wurde mit Natronlauge (2 N, 0,8 - 1,0mL) Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.

Vorreinigung: Das Rohprodukt nach der HCl-Behandlung (vorheriger Schritt) wurde in 1 mL Wasser aufgenommen und auf eine Anionenaustauscherkartusche (Waters, SAX-OH form) gegeben. 80 % der radioaktiven Produkte wurden auf der Kartusche zurückgehalten. Die radioaktiven Produkte wurden mittels wässriger Kochsalzlösung NaCl (0.4 M, 2 ml) von der Kartusche eluiert. Eine Probe wurde mittels HPLC analysiert.

Identifikation durch Radio-HPLC. Pumpe: Gilson 305, Injector: Rheodyne (20 µL Injektionsschleife), Säule: Zorbax -NH₂; 4.6 x 150 mm, mobile Phase: NaH₂PO₄ (10 mM)/Phosphorsäure, pH 3, Fluß: 1 ml/ min, UV-Absorptionsdetektor: Gilson 116 in Serie mit einem Beckman 170 Radiodetektor, UV-Detektion: 210 nm. Rₜ: F-19-Referenz ((rac)-4-F-Glu-hydrochlorid (Diastereomerengemisch: 1/5; beschrieben vide supra)): 12.22 min (UV); Radioaktivitätsdetektion (Beckman): 12,64 min. Ein einzelner radioactiver Peak wurde erhalten, welcher mit der Referenzverbindung ko-eluierte.

HPLC-Reinigung: Pumpe: Gilson 305, Injektor: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioactiver Peak wurde erhalten, welcher mit der Referenzverbindung ko-eluierte. Wird das entstandene Produkt über HPLC aufgereinigt, kann auf die zu vor beschriebene Vorreinigung mittels Anionenaustauscherkartusche verzichtet werden.
Das Produkt konnte mit einer radiochemischen Reinheit von > 90 % und einer Radioaktivität von 15 - 200 mCi (zerfallskorrigiert) erhalten werden.

### Beispiel 2:

### Synthese von [¹⁸F]-(R)-2-Amino-3-fluoro-pentandisäure

Zu einem Gemisch aus 60 µl wässriger 20%iger Tetrabutylammoniumcarbonat-Lösung in 1,5 ml Acetonitril (1.0 ml) wurden [¹⁸F]-fluoridhaltige Lösung (33 µl, 789 MBq) gegeben. Das Lösungsmittel wurde bei 120°C Ofentemperatur im Stickstoffstrom durch Verdampfen entfernt. 1 ml wasserfreies Acetonitril wurde hinzu gegeben und erneut durch Evaporation entfernt. Dieser letzte Schritt wurde nochmals wiederholt. Eine Lösung von 3 mg (S)-2-Benzyloxycarbonylamino-3-(toloyl-sulfonyloxy)-pentan-di-säurediethylester (Chem. Pharm. Bull., 17, 5, (1969), 879-885) in 0,3 ml wasserfreiem Acetonitril wurden zum Rückstand gegeben und gut gerührt. Nach Erhitzen bei 90°C für 15 min wurden 2 ml 40%ige wässrige Bromwasserstoff-Lösung hinzugegeben. Die Reaktionsmischung wurde für 30 min und Überdruck bei 130 °C Ofentemperatur gerührt.

Das Rohprodukt wurde radio-analytisch per HPLC analysiert: Pumpe: Gilson 305, Injector: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. ¹⁹F-Referenz (J. Org. Chem.; 50; 17; (1985); 3163-3167). (UV); Radioaktivitätsdetektion (Beckman). Ein einzelner radioactiver Peak wurde erhalten, welcher mit der Referenzverbindung ko-eluierte.

Die Reinigung der ¹⁸F-markierten Verbindung wurde mittels HPLC-Reinigung durchgeführt: Pumpe: Gilson 305, Injektor: Rheodyne (20 µL Injektionsschleife), Säule: Zorbax - NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioaktiver Peak wurde erhalten, welcher zeitgleich mit der ¹⁹F-Referenzverbindung (J. Org. Chem.; 50; 17; (1985); 3163-3167) eluierte. Wird das entstandene Produkt über HPLC aufgereinigt, kann auf die zu vor beschriebene Vorreinigung mittels Anionenaustauscherkartusche verzichtet werden. Das Produkt konnte mit einer radiochemischen Reinheit von ca. 92 % und einer Radioaktivität von 103 MBq erhalten werden.

### Beispiel 3:

### Di-t-butyl-N-trityl-(S)-glutamat

Zu Di-t-butyl-(S)-glutaminsäurehydrochlorid (20,0 g, 68 mmol, SIGMA, cat#: G-7501), gelöst in MeCl₂ (100 ml), wurden Triethylamin (40 ml) und Tritylchlorid (19,0 g, 68,5 mmol) gegeben. Die Lösung wurde für 24 h bei Raumtemperatur gerührt und anschließend mit gesättigter Natriumkarbonatlösung (3 x) und Wasser (3 x) gewaschen. Die organische Phase wurde über MgSO₄ getrocknet, das Lösungsmittel unter Vakuum entfernt und das entstandene orangefarbene Öl per Flash-Chromatographie an Kieselgel in Hexan/ MeCl₂ (30/ 70) gereinigt. Der gewonnene weiße Feststoff enthielt das Produkt begleitet von einem Rückstand an Trityl-OH. Der Tritylalkohol wurde auskristallisiert durch Lösen des Produktgemisches in einer minimalen Menge an MeCl₂ und Zugabe von Hexan. Nach Filtration und Entfernung des Lösungsmittelgemisches wurde ein farbloses Öl erhalten. Ausbeute: 6,0 g, (18 %). DC: Rf = 0,5 (MeCl₂). Elementaranalyse C₃₂H₃₉NO₄: gefunden C: 76,4; H: 7,6; N: 2,9; berechnet: C: 76,6; H: 7,8; N: 2,8.

### t-Butyl-2-trityl-4-carbo-t-butyloxy 5-hydroxy (2S)-heptanoat

n-Butylithium (5,0 ml, 11 mmol) wurde bei 0 °C zu einer Cyclohexylisopropylaminlösung (3,0 ml, 15 mmol) in Hexan (50 ml) in einem Dreihalskolben gegeben. Die Lösung wurde 30 min. bei 0 °C gerührt, anschließend auf -78 °C abgekühlt und mit Di-t-butyl-N-trityl-(S)-glutamat (5,0 g, 10 mmol) in Hexan (50 ml) versetzt. Der Kolben wurde mit einem Gaseinleitungsrohr versehen und mit einem Vorratsgefäß, welches Paraformaldehyd und einen Gaseinlaß für Argon enthielt, verbunden. Nach der Bildung des Carbanions wurde der Paraformaldehyd auf 180 °C erhitzt und das entstehende Formaldehydgas in einem Argonstrom über 30 min. in das Reaktionsgefäß eingeleitet. Dabei wurde die Badtemperatur bei -78 °C gehalten. Anschließend wurde das Kältebad entfernt die Reaktionsmischung langsam auf Raumtemperatur erwärmt und filtriert um Paraformaldehydrückstände zu entfernen. Das Filtrat wurde in gesättigte wässrige Ammoniumchloridlösung gegeben und mit Diethylether (3 x 250 ml) extrahiert. Die vereinigten organischen Phasen wurden mit Magnesiumsulfat getrocknet und das Lösungsmittel anschließend am Vakuum entfernt. Das entstandene gelbliche Öl wurde mittels Flashchromatographie (Essigester/ MeCl₂ (10/ 90)) gereinigt. Nach Entfernung des Lösungsmittelgemisches wurde ein farbloses Öl erhalten. Ausbeute: 1,2 g, (25 %). DC: Rf = 0,3 (MeCl₂). Elementaranalyse C₃₃H₄₁NO₅: gefunden C: 74,5; H: 7,6; N: 2,8; berechnet: C: 74,6; H: 7,8; N: 2,6.

### t-Butyl-2-trityl-4-carbo-t-butyloxy-5-toluoylsulfonsäure-(2S)-heptanoat

t-Butyl-2-trityl-4-carbo-t-butyloxy-5-hydroxy-(2S)-heptanoat (532 mg, 1,00 mmol) wurde in MeCl₂ (6 ml) und Pyridin (1,2 ml) gelöst. Anschließend wurden *p-*Toluolsulfonylchlorid (118 mg, 0,62 mmol) und Dimethylaminopyridin (13,4 mg, 0,11 mmol) zugegeben und die Reaktionsmischung wurde unter Stickstoffatmosphäre über Nacht bei Raumtemperatur gerührt. Die Reaktionsmischung wurde mit Essigester (3 x) extrahiert, die vereinigten organischen Phasen über Natriumsulfat getrocknet, filtriert und die Lösungsmittel am Rotationsverdampfer entfernt. Das Rohprodukt wurde in wenig MeCl₂ gelöst, auf NH₂-Material aufgezogen und über Säulenchromatographie in Essigester/ Hexan (8:2) gereinigt. Ausbeute: 339 mg, (70 %). Elementaranalyse C₄₀H₄₇NO₇: gefunden C: 70,3; H: 7,1; N: 2,2; S: 5,0 berechnet: C: 70,1; H: 6,9; N: 2,0; S: 4,7.

### t-Butyl-2-trityl-4-carbo-t-butyloxy-5-fluoro-(2S)-heptanoat (HPLC-Referenz)

Wasserfreies Tetrabutylammoniumfluorid (102 mg, 0.4 mmol) wurde zu einer Lösung von t-Butyl-2-trityl-4-carbo-t-butyloxy-5-toluylsulfonsäure-(2S)-heptanoat (54 mg, 0,08 mmol) in wasserfreiem THF (3 ml) gegeben. Die Mischung wurde 4 h am Rückfluß erhitzt. Nach dem die Reaktionsmischung auf Raumtemperatur abgekühlt war, wurde die Mischung mit MeCl₂ versetzt und wässrig extrahiert (3 x). Präparative Dünnschichtchromatographie (MeCl₂/ MeOH (90/10)) lieferte das Produkt als gelbliches Öl. Ausbeute: 22 mg, (51 %). Elementaranalyse C₃₃H₄₀NO_{4:} gefunden C: 74,4; H: 7,7; N: 2,8; berechnet: C: 74,3; H: 7,6; N: 2,6.

### [F-18]-t-Butyl-2-trityl-4-carbo-t-butyloxy 5-fluoro-(2S)-heptanoat (HPLC-Referenz)

[F-18]Fluorid wurde über die ¹⁸O(p,n)¹⁸F-Reaktion in einem Zyklotron hergestellt. Die Isotopenlösung wurde auf eine Sep-Pack Light QMA-Kartusche gegeben und im Luftstrom getrocknet. Das [F-18]Fluorid wurde mit einer Kryptofix 2.2.2/ K₂CO₃-Lösung (22 mg K2.2.2, 4,6 mg K₂CO₃, 2 ml, MeCN (1,77 ml), Wasser (0,23 ml)) von der Kartusche eluiert. Die Lösungsmittel wurden bei 120 °C in einem Argonstrom entfernt. Der Rückstand wurde wurde mit 1 ml wasserfreien MeCN zweimal bei 120 °C in einem Argonstrom azeotrop destilliert. Eine Lösung des Tosylatpräkursors t-Butyl-2-trityl-4-carbo-t-butyloxy-5-toluoylsulfonsäure-(2S)-heptanoat (4 mg) in MeCN (0,2 ml) wurde zu einem Gefäß mit dem getrockneten [F-18]Fluorid gegeben. Die Reaktionsmischung wurde für 10 min. auf 120 °C erhitzt. Anschließend wurde das Lösungsmittel in einem Argonstrom entfernt. Das Lösungsmittel (MeCN) wurde in einem Stickstoffstrom entfernt und der Rückstand mit HCl (6 N, 0.3 - 0.5mL) bei 140 °C für 5 min. behandelt. Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.

Vorreinigung: Das Rohprodukt nach der HCl-Behandlung (vorheriger Schritt) wurde in 1 mL Wasser aufgenommen und auf eine Anionenaustauscherkartusche (Waters, SAX-OH form) gegeben. 80 % der radioaktiven Produkte wurden auf der Kartusche zurückgehalten. Die radioaktiven Produkte wurden mittels wässriger Kochsalzlösung NaCl (0.4 M, 2 ml) von der Kartusche eluiert. Eine Probe wurde mittels HPLC analysiert.

Identifikation durch Radio-HPLC. Pumpe: Gilson 305, Injector: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Rₜ: F-19-Referenz ((rac)-4-F-Glu-hydrochlorid): 14.53 min (UV); Radioaktivitätsdetektion (Beckman): 14,68 min. Ein einzelner radioactiver Peak wurde erhalten, welcher mit der Referenzverbindung ko-eluierte.

HPLC-Reinigung: Pumpe: Gilson 305, Injektor: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioactiver Peak wurde erhalten, welcher mit der Referenzverbindung ko-eluierte. Wird das entstandene Produkt über HPLC aufgereinigt, kann auf die zu vor beschriebene Vorreinigung mittels Anionenaustauscherkartusche verzichtet werden.
Das Produkt konnte mit einer radiochemischen Reinheit von > 90 % und einer Radioaktivität von 20 - 200 mCi (zerfallskorrigiert) erhalten werden.

### Beispiel 4:

### Biologische Charakterisierung:

Zur Evaluierung der Tumoranreicherung und der Gewebeverteilung in Versuchstieren wurde [¹⁸F]-4-Glutaminsäure in einem murinen F9-Teratokarzinom.Modell in NMRI-Nacktmäusen und einem murinen B16F1-Melanom-Modell in C57BI6-Mäusen getestet.
Dazu wurden 1 x 10⁶ Zellen (F9) beziehungsweise 5 x 10⁵ Zellen (B16F1) in 100 µl phosphatgepufferter physiologischer Kochsalzlösung suspendiert und subkutan in der rechten, hinteren Flanke der entsprechenden Versuchstierspezies (NMRI für F9 und C57-B16 für B16F1) inokuliert (Berndorff et al. Clin. Cancer Res. 2005, 11, 2005). Nach 14 Tagen (F9) beziehungsweise 10 Tagen (B16) erreichten die Tumore eine Größe von ca. 80 - 100 mm². [¹⁸F]-4-Glutaminsäure (370 kBq, in 100 µl physiologischer Kochsalzlösung) wurde intravenös in die Schwanzvene appliziert. Nach jeweils 15; 60 und 120 min. wurden die Tiere getötet, Organ- und Tumorgewebe entnommen, gewogen und auf radioaktiven Gehalt vermessen. Die entsprechenden Daten sind in den Tabellen 1 - 4 zusammengefasst.
Die Gewebeverteilungen von [¹⁸F]-4-Glutaminsäure wurden in den gleichen Tumormodellen mit denen von C-14 markierter Glutaminsäure verglichen, wobei 111 kBq [¹⁴C]5-Glutaminsäure intravenös appliziert wurde und die Tiere nach 30, 60 und 240 min (F9) sowie nach 15, 60 und 120 min(B16F1) getötet und analysiert wurden (Tabellen 5-8).

**Tab. 1: Gebewebeaufnahme (% injizierte Dosis pro g Gewebe) von [¹⁸F]4-Glutaminsäure im murinen, embryonalen Teratokarzinom F9 in NMRI-Nacktmäusen nach einmaliger intravenöser Applikation.**

| **Zeitpunkt:** | **0,25 h** | | **1,0 h** | | **2,0 h** | |
|---|---|---|---|---|---|---|
| **%Dosis/g** | | S.D. | | S.D. | | S.D. |
| **Milz** | **2.56** | 0.64 | **0.54** | 0.04 | **0.16** | 0.09 |
| **Leber** | **3.37** | 0.84 | **0.50** | 0.02 | **0.20** | 0.03 |
| **Niere** | **7.24** | 1.33 | **1.30** | 0.17 | **0.37** | 0.12 |
| **Lunge** | **5.08** | 1.19 | **1.32** | 0.24 | **0.48** | 0.10 |
| **Herz** | **2.86** | 0.67 | **0.64** | 0.08 | **0.21** | 0.04 |
| **Hirn** | **0.52** | 0.15 | **0.43** | 0.11 | **0.27** | 0.03 |
| **Fett** | **0.85** | 0.83 | **0.26** | 0.08 | **0.09** | 0.03 |
| **Muskel** | **1.72** | 0.10 | **0.70** | 0.03 | **0.42** | 0.50 |
| ***Tumor*** | ***2.90*** | *0.86* | ***1.75*** | *0.21* | ***1.87*** | *0.38* |
| **Haut** | **2.28** | 0.38 | **0.53** | 0.09 | **0.21** | 0.09 |
| **Blut** | **2.51** | 0.43 | **0.51** | 0.04 | **0.21** | 0.08 |
| **Magen** | **2.56** | 0.62 | **1.26** | 1.29 | **0.33** | 0.16 |
| **Ovar** | **1.34** | 0.45 | **0.41** | 0.16 | **0.12** | 0.05 |
| **Uterus** | **2.54** | 0.48 | **0.72** | 0.19 | **0.32** | 0.18 |
| **Darm** | **2.28** | 0.31 | **1.83** | 0.24 | **1.21** | 0.16 |

**Tab.2: Tumor/ Gewebequotient von [¹⁸F]4-Glutaminsäure im murinen, embryonalen Teratokarzinom F9 in NMRI-Nacktmäusen nach einmaliger intravenöser Applikation.**

| **T/G-Qu.** | | S.D. | | S.D. | | S.D. |
|---|---|---|---|---|---|---|
| **Milz** | **1.12** | 0.13 | **3.22** | 0.16 | **18** | 17.77 |
| **Leber** | **0.86** | 0.18 | **3.48** | 0.29 | **9.23** | 1.32 |
| **Niere** | **0.4** | 0.07 | **1.38** | 0.35 | **5.22** | 0.53 |
| **Lunge** | **0.57** | 0.04 | **1.34** | 0.15 | **4.09** | 1.41 |
| **Herz** | **1.01** | 0.15 | **2.73** | 0.24 | **9.22** | 1.9 |
| **Hirn** | **5.6** | 0.93 | **4.2** | 0.59 | **7.06** | 1.96 |
| **Fett** | **5.85** | 5 | **7.37** | 2.87 | **23.67** | 9.1 |
| **Muskel** | **1.67** | 0.42 | **2.51** | 0.32 | **9.5** | 6.61 |
| **Haut** | **1.25** | 0.23 | **3.4** | 0.95 | **9.4** | 1.74 |
| **Blut** | **1.14** | 0.18 | **3.46** | 0.36 | **9.06** | 1.19 |
| **Magen** | **1.12** | 0.09 | **2.45** | 1.56 | **6.85** | 3.86 |
| **Darm** | **1.26** | 0.27 | **0.97** | 0.18 | **1.54** | 0.11 |
| **Ovar** | **2.43** | 1.22 | **5.05** | 2.93 | **17.74** | 7.63 |
| **Uterus** | **1.18** | 0.46 | **2.59** | 0.92 | **6.96** | 3.18 |

**Tab. 3: Gebewebeaufnahme (% injizierte Dosis pro g Gewebe) von [¹⁸F]4-Glutaminsäure im murinen, syngenen, B16 Melanom in C57BI6-Mäusen nach einmaliger intravenöser Applikation.**

| **Zeitpunkt:** | **0,25 h** | | **1,0 h** | | **2,0 h** | |
|---|---|---|---|---|---|---|
| **%Dosis/g** | | S.D. | | S.D. | | S.D. |
| **Milz** | **4.07** | 0.54 | **0.81** | 0.12 | **0.31** | 0.04 |
| **Leber** | **3.81** | 0.06 | **0.64** | 0.10 | **0.29** | 0.06 |
| **Niere** | **11.35** | 0.66 | **2.25** | 0.97 | **0.57** | 0.13 |
| **Lunge** | **11.10** | 0.60 | **1.47** | 0.34 | **0.56** | 0.16 |
| **Herz** | **3.36** | 0.09 | **0.71** | 0.20 | **0.28** | 0.10 |
| **Hirn** | **0.73** | 0.03 | **0.49** | 0.16 | **0.36** | 0.08 |
| **Fett** | **0.92** | 0.16 | **0.18** | 0.05 | **0.16** | 0.06 |
| **Muskel** | **2.19** | 0.75 | **0.53** | 0.13 | **0.24** | 0.16 |
| ***Tumor*** | ***3.55*** | *0.17* | ***2.14*** | *0.57* | ***1.27*** | *0.24* |
| **Haut** | **2.43** | 0.36 | **0.48** | 0.14 | **0.22** | 0.06 |
| **Blut** | **2.93** | 0.15 | **0.48** | 0.13 | **0.16** | 0.03 |
| **Magen** | **3.41** | 0.28 | **0.59** | 0.20 | **0.30** | 0.05 |
| **Ovar** | **3.57** | 0.79 | **0.58** | 0.17 | **0.45** | 0.19 |
| **Uterus** | **7.17** | 3.27 | **0.69** | 0.20 | **0.76** | 0.56 |
| **Darm** | **3.00** | 0.32 | **1.28** | 0.25 | **1.46** | 0.09 |

**Tab. 4: Tumor/ Gewebequotient von [¹⁸F]4-Glutaminsäure im murinen, syngenen, B16 Melanom in C57BI6-Mäusen nach einmaliger intravenöser Applikation.**

| **T/G-Qu.** | | S.D. | | S.D. | | S.D. |
|---|---|---|---|---|---|---|
| **Milz** | **0.89** | 0.14 | **2.81** | 0.30 | **4.03** | 0.39 |
| **Leber** | **0.93** | 0.05 | **3.63** | 0.57 | **4.42** | 0.31 |
| **Niere** | **0.31** | 0.03 | **1.25** | 0.13 | **2.22** | 0.22 |
| **Lunge** | **0.32** | 0.01 | **1.51** | 0.1 | **2.34** | 0.49 |
| **Herz** | **1.06** | 0.08 | **3.54** | 0.53 | **5.06** | 2.1 |
| **Hirn** | **4.89** | 0.34 | **4.7** | 1.04 | **3.55** | 0.34 |
| **Fett** | **3.95** | 0.81 | **14.16** | 1.1 | **8.85** | 3.19 |
| **Muskel** | **1.72** | 0.44 | **4.3** | 2.47 | **6.95** | 3.5 |
| **Haut** | **1.49** | 0.25 | **5.43** | 1.53 | **5.94** | 0.54 |
| **Blut** | **1.21** | 0.12 | **5.25** | 1.12 | **7.93** | 0.96 |
| **Magen** | **1.05** | 0.12 | **4.02** | 0.96 | **4.24** | 0.63 |
| **Darm** | **1.19** | 0.15 | **1.56** | 0.08 | **0.87** | 0.13 |
| **Ovar** | **1.03** | 0.27 | **4.38** | 0.85 | **3.02** | 0.79 |
| **Uterus** | **0.56** | 0.23 | **3.84** | 1.76 | **2.59** | 2.06 |

Im Vergleich zu diesen Ergebnissen wurde C-14 markierte Glutaminsäure in beiden Tumormodellen untersucht. Die entsprechenden Organverteilungsresultate sind in Tab. 5 - 9 abgebildet.

**Tab. 5: Gebewebeaufnahme (% injizierte Dosis pro g Gewebe) von [¹⁴C]5-Glutaminsäure im murinen, embryonalen Teratokarzinom F9 in NMRI-Nacktmäusen nach einmaliger intravenöser Applikation.**

| **Zeitpunkt:** | **0,5 h** | | **1,0 h** | | **4,0 h** | |
|---|---|---|---|---|---|---|
| **%Dosis/g** | | S.D. | | S.D. | | S.D. |
| **Milz** | **0.78** | 0.12 | **0.55** | 0.06 | **0.52** | 0.03 |
| **Leber** | **1.10** | 0.37 | **0.75** | 0.10 | **0.59** | 0.10 |
| **Niere** | **0.95** | 0.05 | **0.56** | 0.03 | **0.36** | 0.04 |
| **Lunge** | **1.51** | 0.11 | **0.83** | 0.09 | **0.59** | 0.04 |
| **Herz** | **0.87** | 0.08 | **0.49** | 0.09 | **0.18** | 0.01 |
| **Hirn** | **0.11** | 0.01 | **0.08** | 0.01 | **0.05** | 0.01 |
| **Fett** | **0.08** | 0.02 | **0.04** | 0.01 | **0.06** | 0.04 |
| **Muskel** | **0.40** | 0.14 | **0.29** | 0.05 | **0.19** | 0.05 |
| ***Tumor*** | ***0.81*** | *0.32* | ***0.98*** | *0.34* | ***0.64*** | *0.08* |
| **Haut** | **0.77** | 0.24 | **0.55** | 0.08 | **0.41** | 0.13 |
| **Blut** | **0.31** | 0.03 | **0.28** | 0.02 | **0.19** | 0.04 |
| **Magen** | **1.54** | 0.31 | **1.16** | 0.33 | **0.80** | 0.16 |
| **Ovar** | **0.93** | 0.18 | **0.72** | 0.24 | **0.46** | 0.10 |
| **Uterus** | **0.75** | 0.25 | **0.51** | 0.14 | **0.43** | 0.19 |
| **Darm** | **0.81** | 0.32 | **0.93** | 0.31 | **0.68** | 0.14 |

**Tab. 6: Tumor/ Gewebequotient von [¹⁴C]5-Glutaminsäure im murinen, embryonalen Teratokarzinom F9 in NMRI-Nacktmäusen nach einmaliger intravenöser Applikation.**

| **T/G-Qu.** | | | |
|---|---|---|---|
| **Milz** | **1,08** | **1,76** | **1,23** |
| **Leber** | **0,74** | **1,31** | **1,09** |
| **Niere** | **0,85** | **1,74** | **1,81** |
| **Lunge** | **0,53** | **1,17** | **1,10** |
| **Herz** | **0,92** | **1,99** | **3,53** |
| **Hirn** | **7,12** | **12,39** | **13,64** |
| **Fett** | **10,13** | **24,01** | **10,02** |
| **Muskel** | **2,00** | **3,33** | **3,36** |
| **Haut** | **1,04** | **1,77** | **1,58** |
| **Blut** | **2,59** | **3,53** | **3,45** |
| **Magen** | **0,52** | **0,84** | **0,80** |
| **Darm** | **0,99** | **1,05** | **0,94** |
| **Ovar** | **0,86** | **1,35** | **1,40** |
| **Uterus** | **1,08** | **1,93** | **1,50** |

**Tab. 7: Gebewebeaufnahme (% injizierte Dosis pro g Gewebe) von [¹⁴C]5-Glutaminsäure im murinen, syngenen, B16 Melanom in C57BI6-Mäusen nach einmaliger intravenöser Applikation.**

| **Zeitpunkt:** | **0,25 h** | | **1,0 h** | | **2,0 h** | |
|---|---|---|---|---|---|---|
| **%Dosis/g** | | S.D. | | S.D. | | S.D. |
| **Milz** | **1,91** | 0,33 | **1,16** | 0,39 | **0,72** | 0,15 |
| **Leber** | **2,34** | 0,40 | **1,30** | 0,23 | **1,23** | 0,19 |
| **Niere** | **2,97** | 0,54 | **0,99** | 0,16 | **0,92** | 0,04 |
| **Lunge** | **3,74** | 0,99 | **0,87** | 0,09 | **0,83** | 0,09 |
| **Herz** | **3,03** | 0,43 | **0,93** | 0,06 | **0,52** | 0,08 |
| **Hirn** | **0,22** | 0,06 | **0,13** | 0,08 | **0,09** | 0,01 |
| **Fett** | **0,69** | 0,29 | **0,19** | 0,09 | **0,15** | 0,10 |
| **Muskel** | **1,15** | 0,19 | **0,42** | 0,06 | **0,28** | 0,07 |
| ***Tumor*** | ***1,23*** | *0,14* | ***1,03*** | *0,13* | ***0,98*** | *0, 22* |
| **Haut** | **0,86** | 0,16 | **0,32** | 0,15 | **0,28** | 0,19 |
| **Blut** | **0,56** | 0,06 | **0,40** | 0,10 | **0,37** | 0,06 |
| **Magen** | **2,71** | 1,54 | **2,11** | 0,77 | **1,60** | 0,57 |
| **Ovar** | **2,61** | 0,74 | **1,01** | 0,48 | **1,18** | 0,03 |
| **Uterus** | **1,35** | 0,16 | **0,91** | 0,25 | **0,74** | 0,17 |
| **Darm** | **1,81** | 0,58 | **1,47** | 0,60 | **1,38** | 0,32 |

**Tab. 8: Tumor/ Gewebequotient von [¹⁴C]5-Glutaminsäure im murinen, syngenen, B16 Melanom in C57BI6-Mäusen nach einmaliger intravenöser Applikation.**

| **T/G-Qu.** | | | |
|---|---|---|---|
| **Milz** | **0,65** | **0,88** | **1,37** |
| **Leber** | **0,53** | **0,79** | **0,80** |
| **Niere** | **0,42** | **1,03** | **1,07** |
| **Lunge** | **0,33** | **1,18** | **1,18** |
| **Herz** | **0,41** | **1,11** | **1,88** |
| **Hirn** | **5,59** | **7,66** | **10,59** |
| **Fett** | **1,80** | **5,36** | **6,64** |
| **Muskel** | **1,08** | **2,44** | **3,53** |
| **Haut** | **1,44** | **3,23** | **3,47** |
| **Blut** | **2,19** | **2,56** | **2,64** |
| **Magen** | **0,46** | **0,49** | **0,61** |
| **Darm** | **0,68** | **0,70** | **0,71** |
| **Ovar** | **0,47** | **1,01** | **0,83** |
| **Uterus** | **0,91** | **1,13** | **1,33** |

Überraschenderweise zeigt [¹⁸F]4-Glutaminsäure nach intravenöser Injektion (15 min.) eine maximale Tumoranreicherung von 2,90% ID/ g (F9 Teratokarzinom) beziehungsweise 3,55% ID/ g (B16 Melanom) während die maximale Tumoranreicherung zum ähnlich frühen Zeitpunkt (30 min) für das natürliche Substrat [¹⁴C]5-Glutaminsäure nur 0,81% ID/ g beim F9 Teratokarzinom und zum gleichen Zeitpunkt 1,23 beim B16F1 Melanom beträgt.

Auch 1 h nach intravenöser Applikation liegen die Werte mit 1,75% ID/ g beim F9 Teratokarzinom beziehungsweise 2,14% ID/ g beim B16F1 Melanom deutlich höher als für [¹⁴C]5-Glutaminsäure mit nur 0,98% ID/ g (F9 Teratokarzinom) beziehungsweise 1,03% ID/ g (B16F1Melanom).
Die höhere Tumoranreicherung der fluorierten Verbindung verknüpft mit einer raschen Ausscheidung aus physiologisch, vitalem Gewebe/ Organen führt zu einem deutlich verbesserten Tumor/ Hintergrundverhältnis von [¹⁸F]4-Glutaminsäure gegenüber [¹⁴C]5-Glutaminsäure. Der Tumor/ Blutquotient im B16F1 Melanommodell liegt für die fluorierte Verbindung bei 5,3 (1 h p.i.) und 7,9 (2 h p.i.), und ist damit um den Faktor 2 beziehungsweise 3,5 höher als für [¹⁴C]5-Glutaminsäure.
Der Tumor/ Leberquotient, ein wichtiger Parameter für die Eignungsbewertung eines PET-Tracers, liegt für die fluorierte Verbindung im B16F1 Melanommodell bei 3,6 (1 h p.i.) und 4,4 (2 h p.i.), und ist damit um den Faktor 4,6 beziehungsweise 5,5 höher als für [¹⁴C]5-Glutaminsäure. [¹⁸F]4-Glutaminsäure hat deutlich verbesserte pharmakokinetische Eigenschaften verglichen mit dem C-14 substituierten natürlichen Substrat [¹⁴C]5-Glutaminsäure.

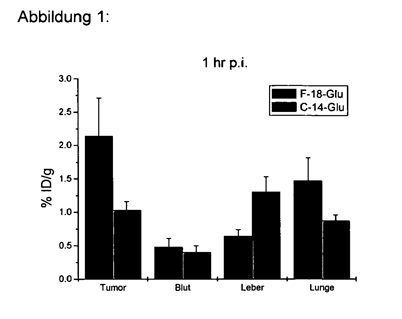

In Abbildung 1 sind die Tumoranreicherung und die Aufnahme in ausgewählten relevanten Organen nach 1 h (a) beziehungsweise 2 h (b) dargestellt.

Abbildung 1 zeigt die Verteilung nach 1 h.

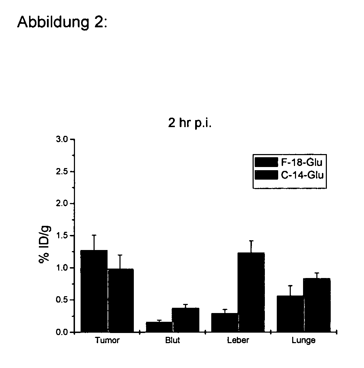

In Abbildung 2 ist die Verteilung nach 2 h dargestellt.

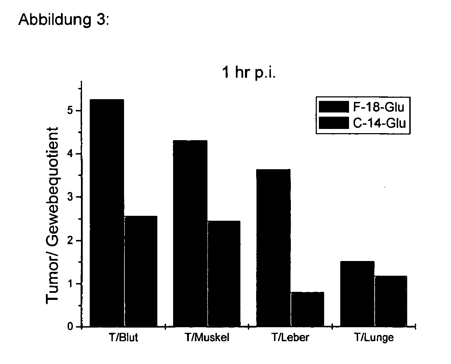

Abbildung 3 zeigt die resultierenden Gewebequotienten inklusive Tumor/ Muskelgewebe nach 1 h.

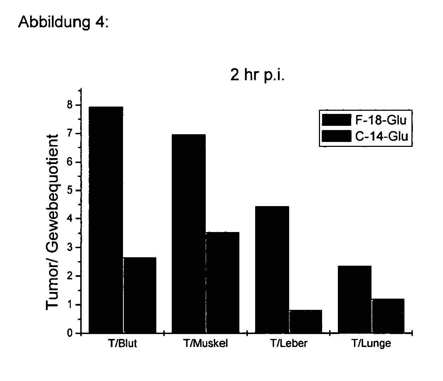

Abbildung 4 zeigt die resultierenden Gewebequotienten inklusive Tumor/ Muskelgewebe nach 2h.

## Patentansprüche

1. Verbindungen der allgemeinen Formel I 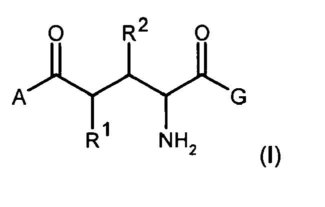 worin
A für
a) Hydroxy
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl
e) N(C₁-C₅ Alkyl)₂,
f) NH₂,
g) N(H)-L,
h) O-L oder
i) O-Z
steht,
G für
a) OH
b) verzweigtes oder unverzweigtes O-C₁-C₅ Alkyl
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl oder
e) verzweigtes oder unverzweigtes O-C₂-C₅ Alkinyl,
steht,
R¹ und R² für
a) Wasserstoff
b) ¹⁸F
c) verzweigtes oder unverzweigtes ¹⁸F-C₁-C₅ Alkoxy
d) verzweigtes oder unverzweigtes ¹⁸F-C₁-C₅ Alkyl,
e) verzweigtes oder unverzweigtes ¹⁸F-hydroxy-C₁-C₅ Alkyl,
f) verzweigtes oder unverzweigtes ¹⁸F-C₂-C₅ Alkenyl, oder
g) verzweigtes oder unverzweigtes ¹⁸F-C₂-C₅ Alkinyl,
h) Hydroxyl,
i) verzweigtes oder unverzweigtes C₁-C₅ Alkyl oder
j) verzweigtes oder unverzweigtes C₁-C₅ Alkoxy
steht,
mit der Maßgabe, dass einer der Substituenten R¹ oder R² genau ein ¹⁸F Isotop und der jeweils andere Substituent kein ¹⁸F Isotop beinhaltet,
L für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl,
steht, und
Z für ein Metallion steht,
wobei
n = 0, 1, 2 oder 3 ist.

2. Verbindungen nach Anspruch 1, **dadurch gekennzeichnet, dass** A für OH, methoxy oder NH₂ steht.

3. Verbindung nach Ansprüchen 1 oder 2, **dadurch gekennzeichnet, dass** A für OH steht.

4. Verbindung nach Ansprüchen 1 oder 2, **dadurch gekennzeichnet, dass** A für NH₂ steht.

5. Verbindungen nach Ansprüchen 1 bis 4, **dadurch gekennzeichnet, dass** R¹ und R² ausgewählt ist aus der Gruppe Wasserstoff, ¹⁸F, ¹⁸F-methoxy, ¹⁸F-ethoxy, ¹⁸F-propoxy, ¹⁸F-methyl, ¹⁸F-ethyl und ¹⁸F-propyl mit der Maßgabe, dass einer der Substituenten R¹ oder R² genau ein ¹⁸F Isotop beinhaltet und der jeweils andere Substituent Wasserstoff ist.

6. Verbindung nach Ansprüchen 1 bis 4, **dadurch gekennzeichnet, dass** R¹ ausgewählt ist aus der Gruppe Hydroxyl, CH₃, C₂H₅ und C₃H₇ , und R² für ¹⁸F steht.

7. Verbindung nach Ansprüchen 1 bis 4, **dadurch gekennzeichnet, dass** R² ausgewählt ist aus der Gruppe Hydroxyl, CH₃, C₂H₅ und C₃H₇ , und R¹ für ¹⁸F steht.

8. Verbindung nach Ansprüchen 1 bis 4, **dadurch gekennzeichnet, dass** R¹ für ¹⁸F und R² für Wasserstoff steht.

9. Verbindung nach Ansprüchen 1 bis 4, **dadurch gekennzeichnet, dass** R² für ¹⁸F und R¹ für Wasserstoff steht.

10. Verbindung nach Ansprüchen 1 bis 9, **dadurch gekennzeichnet, dass** G ausgewählt ist aus der Gruppe OH, Methoxy oder Ethoxy.

11. Verbindung nach Ansprüchen 1 bis 10, **dadurch gekennzeichnet, dass** Z ausgewählt ist aus der Gruppe Mg²⁺, Ca²⁺, Na⁺ und K⁺

12. Verbindung nach Ansprüchen 1 bis 11, **dadurch gekennzeichnet, dass** Z gleich Ni²⁺ ist.

13. Verbindung nach Anspruch 1 ausgewählt aus der Gruppe von Verbindungen mit der Formel:

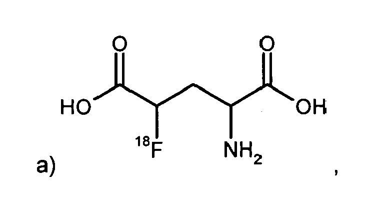

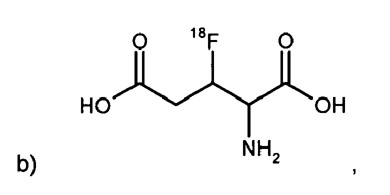

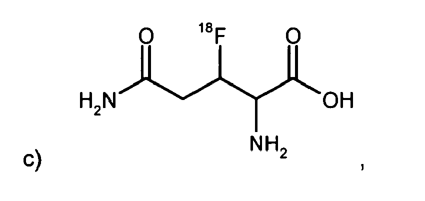

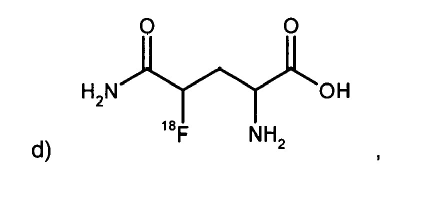

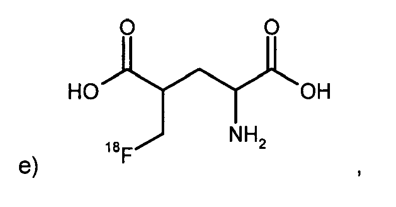

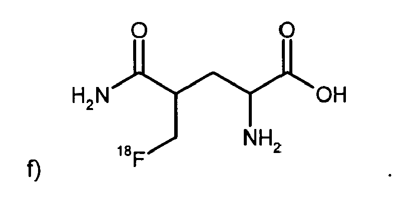

14. Verbindungen der allgemeinen Formel (II): 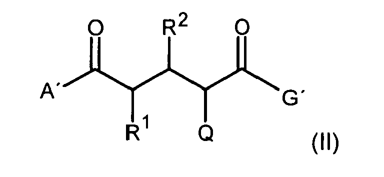 worin,
A' für
a) Hydroxy
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-U
h) N(H)-L', oder
i) O-L'
steht,
G' für
a) hydroxyl
b) O-Z'
c) verzweigtes oder unverzweigtes O-C₁-C₅ Alkyl
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl, oder
f) verzweigtes oder unverzweigtes O-C₂-C₅ Alkinyl
steht,
R¹ und R² für
a) Wasserstoff
b) ¹⁸F
c) verzweigtes oder unverzweigtes ¹⁸F C₁-C₅ Alkoxy
d) verzweigtes oder unverzweigtes ¹⁸F-C₁-C₅ Alkyl,
e) verzweigtes oder unverzweigtes ¹⁸F-C₁-C₅-hydroxy-alkyl,
f) verzweigtes oder unverzweigtes ¹⁸F-C₂-C₅ Alkenyl,
g) verzweigtes oder unverzweigtes ¹⁸F-C₂-C₅ Alkinyl,
h) Hydroxyl,
i) verzweigtes oder unverzweigtes C₁-C₅ Alkyl, oder
j) verzweigtes oder unverzweigtes C₁-C₅ Alkoxy
steht,
mit der Maßgabe, dass genau einer der Substituenten R¹ oder R² genau ein ¹⁸F Isotop und der jeweils andere Substituent kein ¹⁸F Isotop beinhaltet,
Q für
a) N(H)-tert-Butoxycarbonyl,
b) N(H)-Allyloxycarbonyl,
c) N(H)-Benzyloxycarbonyl,
d) N(H)-Ethoxycarbonyl
e) N(H)-Methoxycarbonyl
f) N(H)-Propoxycarbonyl
e) N(H)-2,2,2-Trichlorethoxycarbonyl
f) N(H)-1,1-Dimethylpropinyl
g) N(H)-1-Methyl-1-phenyl-ethoxycarbonyl
h) N(H)-1-Methyl-1-(4-biphenylyl)-ethoxycarbonyl
i) N(H)-cyclobutylcarbonyl
j) N(H)-1-Methylcyclobutylcarbonyl
k) N(H)-Vinylcarbonyl
l) N(H)-Allylcarbonyl
m) N(H)-Adamantylcarbonyl
n) N(H)-Diphenylmethylcarbonyl
o) N(H)-Cinnamylcarbonyl
p) N(H)-Formyl
q) N(H)-Benzoyl
r) N(H)-Trityl
s) N(H)-p-Methoxyphenyl-diphenylmethyl
t) N(H)-di-(p-Methoxyphenyl)-phenylmethyl, oder
u) 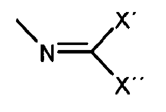
v) N-(tert-Butoxycarbonyl)₂,
steht,
L' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
U für
a) tert-Butoxycarbonyl,
b) Allyloxycarbonyl,
c) Benzyloxycarbonyl,
d) Ethoxycarbonyl,
e) Methoxycarbonyl oder
f) Propoxycarbonyl
steht,
X und X' unanhängig voneinander für
a) verzweigtes oder unverzweigtes C₁-C₅ Alkyl,
b) substituiertes oder unsubstituiertes Aryl oder
c) Aralkyl
steht, und
Z' für ein Metallion
steht,
wobei n = 0, 1, 2 oder 3 ist.

15. Verbindungen nach Anspruch 14, **dadurch gekennzeichnet, dass** A' für OH, verzweigtes oder unverzweigtes C₁-C₅ Alkoxy, -NH-tert-Butoxycarbonyl oder NH₂ steht.

16. Verbindungen nach Ansprüchen 14 oder 15, **dadurch gekennzeichnet, dass** A' für Ethoxy steht.

17. Verbindungen nach Ansprüchen 14 bis 16, **dadurch gekennzeichnet, dass** A' für NH₂ steht.

18. Verbindungen nach Ansprüchen 14 bis 17, **dadurch gekennzeichnet, dass** R¹ und R² ausgewählt ist aus der Gruppe Wasserstoff, ¹⁸F, ¹⁸F-methoxy, ¹⁸F-ethoxy, ¹⁸F-propoxy, ¹⁸F-methyl, ¹⁸F-ethyl und ¹⁸F-propyl mit der Maßgabe, dass einer der Substituenten R¹ oder R² genau ein ¹⁸F Isotop beinhaltet und der jeweils andere Substituent Wasserstoff ist.

19. Verbindung nach Ansprüchen 14 bis 17, **dadurch gekennzeichnet, dass** R¹ ausgewählt ist aus der Gruppe Hydroxyl, CH₃, C₂H₅ und C₃H₇ , und R² für ¹⁸F steht.

20. Verbindung nach Ansprüchen 14 bis 17, **dadurch gekennzeichnet, dass** R² ausgewählt ist aus der Gruppe Hydroxyl, CH₃, C₂H₅ und C₃H₇, und R¹ für ¹⁸F steht.

21. Verbindung nach Ansprüchen 14 bis 17, **dadurch gekennzeichnet, dass** R¹ für ¹⁸F und R² für Wasserstoff steht.

22. Verbindung nach Ansprüchen 14 bis 17, **dadurch gekennzeichnet, dass** R² für ¹⁸F und R¹ für Wasserstoff steht.

23. Verbindung nach Ansprüchen 14 bis 22, **dadurch gekennzeichnet, dass** G' ausgewählt ist aus der Gruppe OH, Ethoxy, Methoxy und OZ'.

24. Verbindung nach Ansprüchen 14 bis 23, **dadurch gekennzeichnet, dass** G' für Ethoxy steht.

25. Verbindung nach Ansprüchen 14 bis 24, **dadurch gekennzeichnet, dass** Z' ausgewählt ist aus der Gruppe Na⁺, K⁺, Ca²⁺ und Mg²⁺.

26. Verbindung nach Ansprüchen 14 bis 25, **dadurch gekennzeichnet, dass** Z' Ni²⁺ ist.

27. Verbindung nach Ansprüchen 14 bis 26, **dadurch gekennzeichnet, dass** Q ausgewählt ist aus der Gruppe N(H)-tert-Butoxycarbonyl, N(H)-Benzyloxycarbonyl und

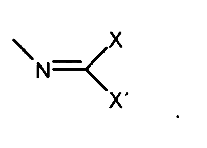

28. Verbindung nach Ansprüchen 14 bis 27 **dadurch gekennzeichnet, dass** Q für N(H)-tert-Butoxycarbonyl steht.

29. Verbindung nach Ansprüchen 14 bis 28 **gekennzeichnet dadurch, dass** Q für 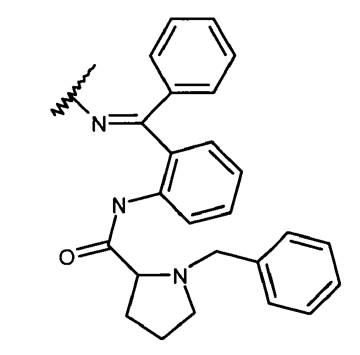 steht.

30. Verbindung nach Anspruch 14 ausgewählt aus der Gruppe von Verbindungen mit der Formel:

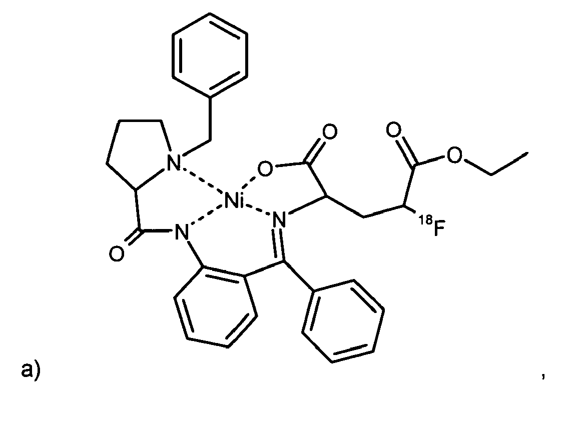

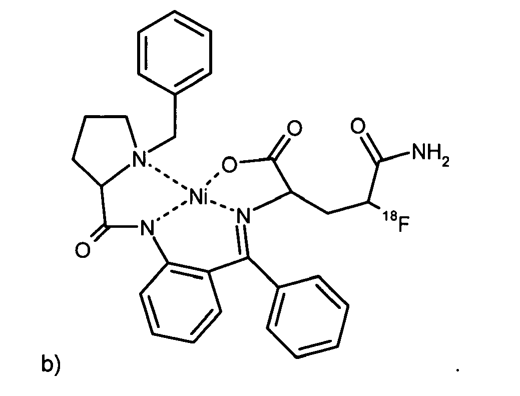

31. Verfahren zur Herstellung von Verbindungen der allgemeinen Formel (I) gemäß Ansprüchen 1 bis 13.

32. Verfahren zur Herstellung von Verbindungen der allgemeinen Formel (II) gemäß Ansprüchen 14 bis 30.

33. Verbindungen gemäß den Ansprüchen 1 bis 30 zur Verwendung als Arzneimittel.

34. Verbindungen gemäß den Ansprüchen 1 bis 30 zur Verwendung in der Diagnose von Tumorerkrankungen.

35. Verwendung von Verbindungen gemäß den Ansprüchen 1 bis 30 zur Herstellung eines Arzneimittels für die Diagnose von Tumorerkrankungen.

36. Verbindungen der Formel (III) 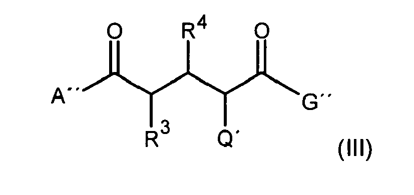 worin
A" für
a) Hydroxy
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl
e) N(C₁-C₅ Alkyl)₂,
f) NH₂,
g) N(H)-U'
h) N(H)-L" oder
i) O-L"
steht,
G" für
a) hydroxyl
b) O-Z"
c) verzweigtes oder unverzweigtes O-C₁-C₅ Alkyl
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl,
f) verzweigtes oder unverzweigtes O-C₂-C₅ Alkinyl, oder
g) triphenylmethoxy
steht,
R³ und R⁴ für
a) Wasserstoff
j) verzweigtes oder unverzweigtes E-C₁-C₅ Alkoxy
k) verzweigtes oder unverzweigtes E-C₁-C₅ Alkyl,
l) verzweigtes oder unverzweigtes E-C₁-C₅ Hydroxy-alkyl,
m) verzweigtes oder unverzweigtes E-C₂-C₅ Alkenyl,
n) verzweigtes oder unverzweigtes E-C₂-C₅ Alkinyl,
o) Hydroxy
p) verzweigtes oder unverzweigtes C₁-C₅ Alkyl, oder
q) verzweigtes oder unverzweigtes C₁-C₅ Alkoxy
steht, mit der Maßgabe, dass genau einer der Substituenten R¹ oder R² ein E beinhaltet und der jeweils andere Substituent kein E beinhaltet,
E für
a) Chloro,
b) Bromo,
c) Mesyloxy
d) Trifluormesyloxy
e) Nonafluorbutyloxy, oder
f) Tosyloxy
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl
e) N(H)-Methoxycarbonyl
f) N(H)-Propoxycarbonyl
g) N(H)-2,2,2-Trichlorethoxycarbonyl
h) N(H)-1,1-Dimethylpropinyl
i) N(H)-1-Methyl-1-phenyl-ethoxycarbonyl
j) N(H)-1-Methyl-1-(4-biphenylyl)-ethoxycarbonyl
k) N(H)-Cyclobutylcarbonyl
l) N(H)-1-Methylcyclobutylcarbonyl
m) N(H)-Vinylcarbonyl
n) N(H)-Allylcarbonyl
o) N(H)-Adamantylcarbonyl
p) N(H)-Diphenylmethylcarbonyl
q) N(H)-Cinnamylcarbonyl
r) N(H)-Formyl
s) N(H)-Benzoyl
t) N(H)-Trityl
u) N(H)-p-Methoxyphenyl-diphenylmethyl
v) N(H)-di-(p-Methoxyphenyl)-phenylmethyl, oder
w) 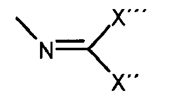
x) N-(tert-Butoxycarbonyl)₂,
steht,
L" für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
U' für
a) tert-Butoxycarbonyl,
b) Allyloxycarbonyl,
c) Benzyloxycarbonyl, oder
d) Ethoxycarbonyl
steht,
X" and X"' unanhängig voneinander für
a) verzweigtes oder unverzweigtes C₁-C₅ Alkyl,
b) substituiertes oder unsubstituiertes Aryl
c) Alkylaryl oder
d) Heteroaryl
steht, und
Z" für ein Metallkation steht,
wobei n = 0, 1 oder 2 ist.

37. Verwendung von Verbindungen der Formel (IV) zur Herstellung von Verbindungen der Formel (I) oder (II): 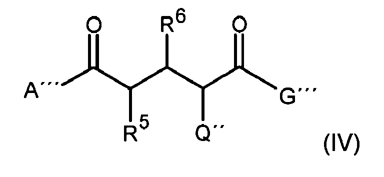 worin
A"' für
a) Hydroxy
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl
e) N(C₁-C₅ Alkyl)₂,
f) NH₂,
g) N(H)-U"
h) N(H)-L'" oder
i) O-L"'
steht,
G'" für
a) hydroxyl
b) O-Z"
c) verzweigtes oder unverzweigtes O-C₁-C₅ Alkyl
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl oder
f) verzweigtes oder unverzweigtes O-C₂-C₅ Alkinyl,
g) triphenylmethoxy
steht,
R⁵ und R⁶ für
c) Wasserstoff oder
d) E'
steht, mit der Maßgabe, dass genau einer der Substituenten R⁵ oder R⁶ ein E' beinhaltet und der jeweils andere Substituent kein E' beinhaltet,
E' für
a) Chloro,
b) Bromo,
c) Mesyloxy
d) Trifluormesyloxy
e) Nonafluorbutyloxy oder
f) Tosyloxy
steht,
Q" für
a) N(H)-tert-Butoxycarbonyl,
b) N(H)-Allyloxycarbonyl,
c) N(H)-Benzyloxycarbonyl,
d) N(H)-Ethoxycarbonyl
e) N(H)-Methoxycarbonyl
f) N(H)-Propoxycarbonyl
g) N(H)-2,2,2-Trichlorethoxycarbonyl
h) N(H)-1,1-Dimethylpropinyl
i) N(H)-1-Methyl-1-phenyl-ethoxycarbonyl
j) N(H)-1-Methyl-1-(4-biphenylyl)-ethoxycarbonyl
k) N(H)-cyclobutylcarbonyl
l) N(H)-1-Methylcyclobutylcarbonyl
m) N(H)-Vinylcarbonyl
n) N(H)-Allylcarbonyl
o) N(H)-Adamantylcarbonyl
p) N(H)-Diphenylmethylcarbonyl
q) N(H)-Cinnamylcarbonyl
r) N(H)-Formyl
s) N(H)-Benzoyl
t) N(H)-Trityl
u) N(H)-p-Methoxyphenyl-diphenylmethyl
v) N(H)-di-(p-Methoxyphenyl)-phenylmethyl, oder
w) 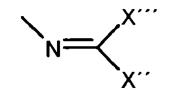
x) N-(tert-Butoxycarbonyl)₂,
steht,
L"' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
U" für
a) tert-Butoxycarbonyl,
b) Allyloxycarbonyl,
c) Benzyloxycarbonyl, oder
d) Ethoxycarbonyl
steht,
X" and X"' unanhängig voneinander für
a) verzweigtes oder unverzweigtes C₁-C₅ Alkyl,
b) substituiertes oder unsubstituiertes Aryl
c) Alkylaryl, oder
d) Heteroaryl
steht, und
Z" für ein Metallkation steht,
wobei n = 0, 1 oder 2 ist.
